# EUROPÄISCHE PATENTSCHRIFT

(11) **EP 1 181 395 B1**
(45) Veröffentlichungstag und Bekanntmachung des Hinweises auf die Patenterteilung: **12.04.2006**
(21) Anmeldenummer: 00945609.6
(22) Anmeldetag: 07.06.2000
(51) Int. Cl.: C12Q 1/68, C12N 15/10, A61K 48/00

(54) **VERFAHREN ZUR SYNTHESE VON DNA-FRAGMENTEN**
METHOD FOR THE SYNTHESIS OF DNA FRAGMENTS
PROCEDE POUR LA SYNTHESE DE FRAGMENTS D'ADN

(30) Priorität: 07.06.1999 DE 19925862
(43) Veröffentlichungstag der Anmeldung: 27.02.2002
(73) Patentinhaber: Sloning BioTechnology GmbH, 85250 Altomünster (DE)
(72) Erfinder: SCHATZ, Octavian, D-85250 Altomünster (DE)
(74) Vertreter: Bohmann, Armin K.
(86) Internationale Anmeldenummer: PCT/DE2000/001863
(87) Internationale Veröffentlichungsnummer: WO 2000/075368

(56) Entgegenhaltungen:
- WO-A-93/19202
- WO-A-95/17413
- WO-A-98/10095
- WO-A-99/47536
- EUGEN UHLMANN: "An alternative approach in gene synthesis: use of long selfpriming oligodeoxynucleotides for the construction of double-stranded DNA" GENE, Bd. 71, 15. November 1988 (1988-11-15), Seiten 29-40, XP000941756
- WLODEK MANDECKI ET AL.: "A totally synthetic plasmid for general cloning, gene expression and mutagenesis in Escherichia coli" GENE, Bd. 94, 28. September 1990 (1990-09-28), Seiten 103-107, XP000941757

### SEQUENZPROTOKOLL

<110> Diavir GmbH
<120> Verfahren zur Synthese von DNA-Fragmenten
<130> DV-001 PCT
<140> xx
   <141> 2000-06-07
<150> DE 199 25 862.7
   <151> 1999-06-07
<160> 7
<170> PatentIn Ver. 2.1
<210> 1
   <211> 26
   <212> DNA
   <213> Künstliche Sequenz
<220>
   <223> Beschreibung der künstlichen Sequenz:Oligonukleotid
<400> 1
   gcttcgagac gcgttttcgc gtctcg 26
<210> 2
   <211> 32
   <212> DNA
   <213> Künstliche Sequenz
<220>
   <223> Beschreibung der künstlichen Sequenz:Oligonukleotid
<400> 2
   agaatggtct tcgagctttt gctcgaagac ca 32
<210> 3
   <211> 16
   <212> DNA
   <213> Künstliche Sequenz
<220>
   <223> Beschreibung der künstlichen Sequenz:Oligonukleotid
<400> 3
   cgcggatccg cggcgt 16
<210> 4
   <211> 20
   <212> DNA
   <213> Künstliche Sequenz
<220>
   <223> Beschreibung der künstlichen Sequenz:Oligonukleotid
<400> 4
   cgagacgccg cggatccgcg 20
<210> 5
   <211> 34
   <212> DNA
   <213> Künstliche Sequenz
<220>
   <223> Beschreibung der künstlichen Sequenz:Oligonukleotid
<400> 5
   aagcttctgg agaccgcttt tgcggtctcc agaa 34
<210> 6
   <211> 20
   <212> DNA
   <213> Künstliche Sequenz
<220>
   <223> Beschreibung der künstlichen Sequenz:Oligonukleotid
<400> 6
   ctcgaagcgg agaccgccac 20
<210> 7
   <211> 16
   <212> DNA
   <213> Künstliche Sequenz
<220>
   <223> Beschreibung der künstlichen Sequenz:Oligonukleotid
<400> 7
   gtggcggtct ccgctt 16

## Beschreibung

Die vorliegende Erfindung betrifft ein Verfahren zur Herstellung eines Nukleinsäuremoleküls und einen Kit zur Herstellung einer Nukleinsäuresequenz.

Nach dem derzeitigen Stand der Technik müssen für eine Synthese einer etwa 2,5 kb großen Nukleinsäuresequenz zunächst etwa 50 verschiedene, teilweise überlappende ca. 80mer Oligonukleotide synthetisiert und aufgereinigt werden. Diese werden dann paarweise oder in Subsets hybridisiert und mittels einer Klenow-Polymerase-Reaktion aufgefüllt oder mit den außen liegenden Oligonukleotiden als Primer in einer Polymerasekettenreaktion (PCR) hergestellt und (meist über einzubauende Restriktioosstellen) unidirektional miteinander verknüpft. Dieses Verfahren ist als "Gap filling" Methode bekannt. Alternativ lassen sich Genfragmente durch enzymatische oder chemische Ligation synthetisieren; diese Fragmente können dann nach Aufreinigung und/oder Klonierung zu größeren Genabschnitten zusammengesetzt werden (sog. Cassettenmethode). Beide Prozeduren erfordern im Idealfall mindestens eine Woche, im Regelfall jedoch eher 6-12 Wochen, mitunter sogar 6 Monate. Sequenzielle, an Festphasen gebundene Verfahren haben wegen der Vielzahl der notwendigen Reaktionsschritte nur geringe Ausbeuten und sind daher auch sehr fehleranfällig.

Eines der Hauptprobleme besteht darin, daß längere Oligonukleotide aus Gründen der Kopplungseffizienz, die selbst bei gut verlaufenden Synthesen nur 99% pro Schritt erreicht, immer einen unvermeidbaren Anteil an Abbruchprodukten aufweisen. Darüber hinaus kommt es auch zu Deletionen, die aus nicht 100%igem Capping resultieren. Selbst bei sehr guten Synthesen liegt dieser Anteil bei ca. 0,25% pro Kopplungsschritt. Auch die Abtrennung der Tritylschutzgruppen nach Beendigung der Synthese verläuft nicht vollständig. Die so entstandenen unvollständigen Oligonukleotidprodukte können selbst mit großem Aufwand von längeren Oligonukleotiden nicht vollständig abgetrennt werden.

Bei einer durchschnittlichen Kopplungseffizienz von 98% erhält man beispielsweise bei einem 80mer eine Ausbeute des gewünschten Produkts mit voller Länge von lediglich 19.86%. Mit den heutzutage verfügbaren Aufreinigungsverfahren kann das gewünschte Endprodukt in einer Reinheit von bestenfalls 95% dargestellt werden. Auch wenn dann nur noch ein kleiner Teil der endgereinigten Oligonukleotide fehlerhaft ist, steigt dennoch die Wahrscheinlichkeit einer fehlerhaften Endsequenz mit der Zahl der eingesetzten Oligonukleotide dramatisch an. Eine Sequenz, die sich aus 50 der beschriebenen Oligonukleotide zusammensetzt, ist demnach nur in 7,7% aller Fälle korrekt und muss deshalb in aller Regel nachgearbeitet werden. Ein relativ seltener Einbau falscher Basen aufgrund von Fehlkopplungen während der Synthese ist dabei nicht berücksichtigt.

Auf Grund der Vielfalt möglicher Sequenzen selbst relativ kurzer Oligonukleotide (bereits von einem 30mer existieren über 10¹⁸ mögliche Sequenzvarianten) ist es zudem praktisch unmöglich, Oligonukleotide für verschiedene Genkonstrukte wieder zu verwenden. Daher ist es technisch nicht machbar, sämtliche zur Generierung beliebiger Sequenzen benötigte Oligonukleotide vorzuhalten. Für jedes neue Genkonstrukt müssen jeweils neue Oligonukleotide synthetisiert und aufgereinigt werden. Nur ein Bruchteil des synthetisierten Materials wird jedoch tatsächlich für die Gensynthese eingesetzt, der Rest kann aus oben beschriebenen Gründen nicht verwertet werden. Die nicht gelöste Einbindung der Oligonukleotidsynthese und deren Aufreinigung in den Gensyntheseprozess ist eines der Haupthindernisse, weswegen eine vollständige Automatisierung dieses Prozesses derzeit technisch nur äußerst schwierig und praktisch wahrscheinlich überhaupt nicht zu realisieren.

WO95/17413 offenbart ein Verfahren zur Herstellung oligomerer oder polymerer Funktionselemente, die durch Verknüpfung von mindestens zwei Formenelementen unter Einsatz einer festen Phase als Reaktionsträger generiert werden. Dabei unterbleibt eine Umsetzung der durch einen Zyklus aus Ligation und Restriktion hergestellten Oligonukleotide miteinander.

WO99/47536, welches ein Dokument gemäß Artikel 54(3,4) EPÜ darstellt und somit nur für die Frage der Neuheit zu berücksichtigen ist, offenbart ein Verfahren zur Herstellung einer Nukleinsäure unter Verwendung eines Restriktionsenzyms vom Typ IIS.

Die der vorliegenden Erfindung zugrundeliegende Aufgabe besteht somit in der Bereitstellung eines Verfahrens zur effizienten Synthese doppelsträngiger DNA-Fragmente beliebiger Sequenz und Länge. Eine weitere Aufgabe besteht darin, eine Methode bereit zu stellen, welche es erlaubt, beliebige DNA-Moleküle aus einer begrenzten Bibliothek von Grundbausteinen zusammen zu setzen. Eine weitere Aufgabe besteht darin, ein Verfahren aufzuzeigen, welches die **parallele** Synthese und die **sequenzunabhängige** Verknüpfung beliebiger Genfragmente gestattet. Die Erfüllung dieser beiden Voraussetzungen ist notwendig für die Realisierung einer kompletten Automatisierung des Gensyntheseverfahrens. Eine weitere Aufgabe besteht in der Bereitstellung eines Kits zur automatisierten Herstellung doppelsträngiger DNA-Fragmente.

Die Aufgabe wird gelöst durch den Gegenstand der unabhängigen Ansprüche. Bevorzugte Ausführungsformen ergeben sich aus den Unteransprüchen.

In einer weiteren Ausführungsform des erfindungsgemäßen Kits ist vorgesehen, dass als Enzyme eine Ligase oder Topoisomerase und/oder ein oder mehrere Restriktionsenzym(e) und/oder eine Exonuklease und/oder eine Phosphatase umfassen.

Die Erfindung wird weiter durch die folgenden Figuren erläutert.

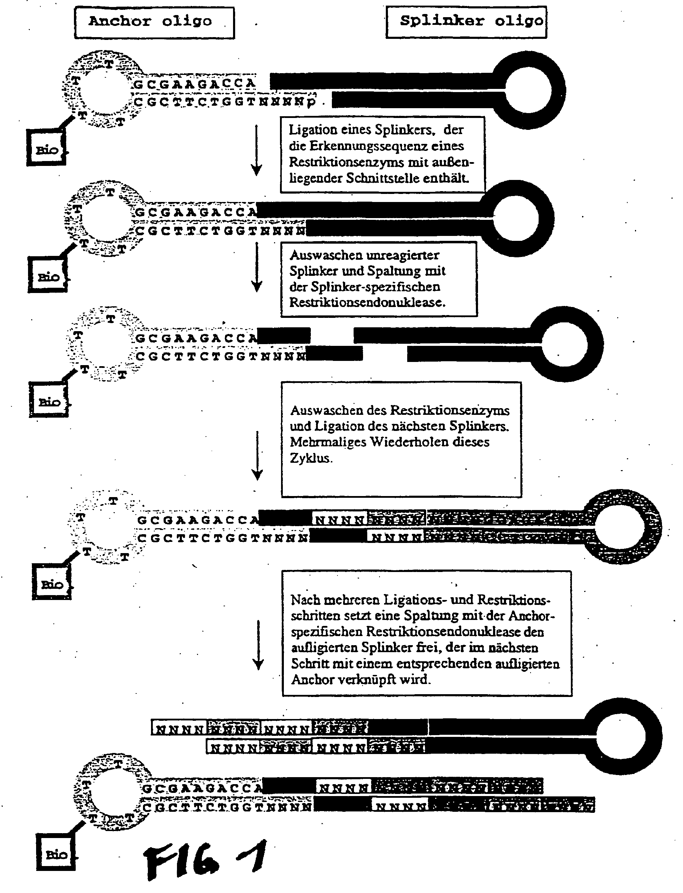

Figur 1 zeigt eine schematische Darstellung des erfindungsgemäßen Verfahrens. Bio bedeutet eine Modifikation (z.B. Biotin), mit der das Anchor-Oligonukleotid an eine feste Matrix (z.B. Streptavidin) gekoppelt ist. T, G, C, A und N bezeichnen die Nukleinsäurebasen, wobei T Thymidin, G Guanin, C Cytosin, A Adenin und N eine beliebige der vier Nukleinsäurebasen bedeutet.

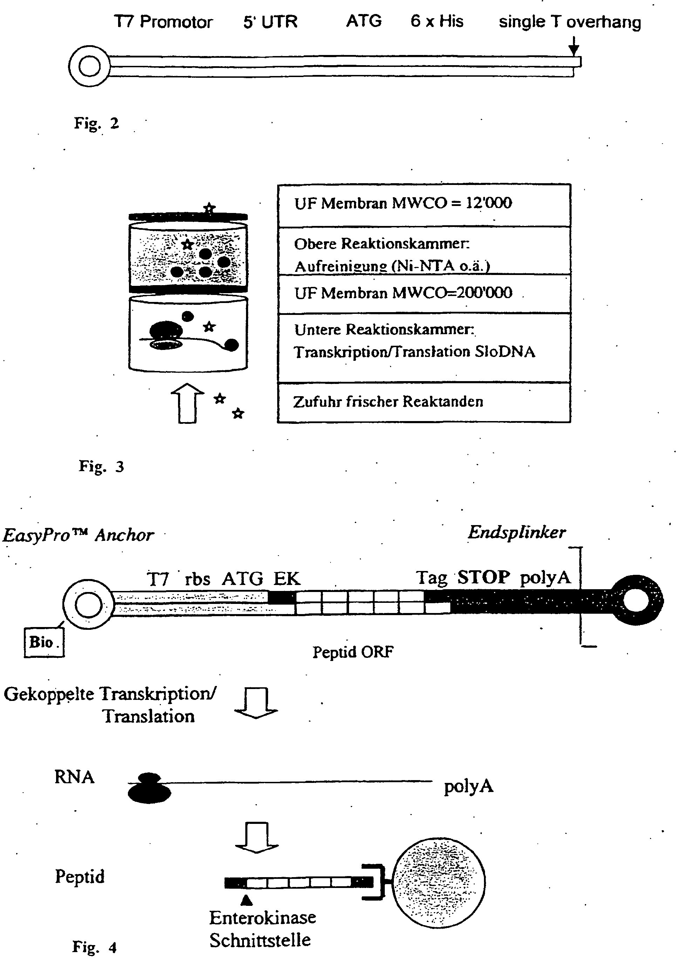

Figur 2 zeigt schematisch den Aufbau eines EasyPro™ Transkriptions/Translationssystems von PCR-Fragmenten. Bio bedeutet eine Modifikation, mit der das Anchor-Oligonukleotid an eine feste Matrix gekoppelt ist. 5'-UTR bedeutet 5'-untranslatierter Bereich. ATG bedeutet Startcodon. 6 x His bedeutet eine sechsmalige Aneinanderreihung von Histidincodons. Single T overhang bedeutet einen Überhang von einem Thymidinrest.
Figur 3 zeigt eine schematische Darstellung eines Minireaktors für die Proteinsynthese.
Figur 4 zeigt eine schematische Darstellung der Produktion einer Peptidlibrary mit dem QuickPep™-Verfahren. Bio bedeutet eine Modifikation, mit der das Anchor-Oligonukleotid an eine feste Matrix gekoppelt ist. T7 bedeutet T7-Promotor. rbs bedeutet interne Ribosomenbindungsstelle. ATG bedeutet Startcodon. EK bedeutet Enterokinase-Schnittstelle. Peptid ORF bedeutet den offenen Leserahmen des Peptids. STOP bedeutet das Stopcodon. Poly A bezeichnet den poly-A-Schwanz.

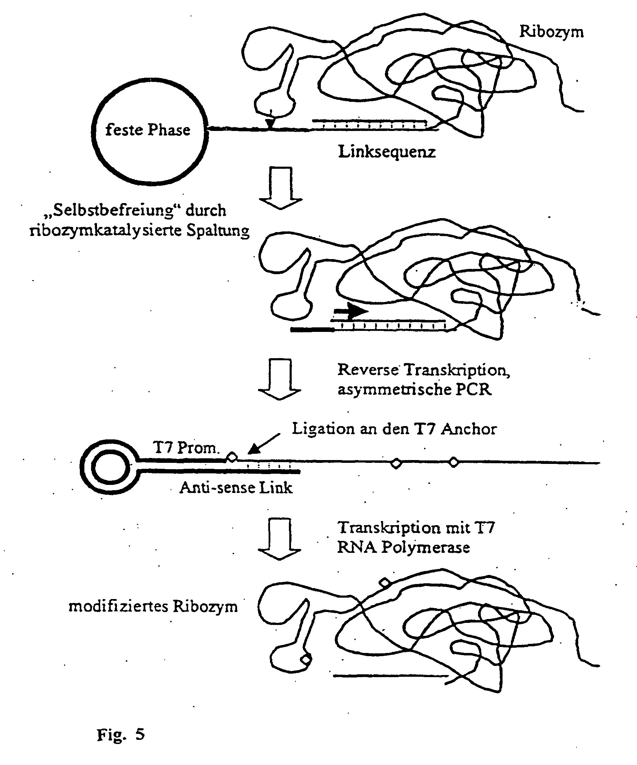

Figur. 5 zeigt eine schematische Darstellung der Selektion von Ribozymen mit dem RiboSelect™-Verfahren.

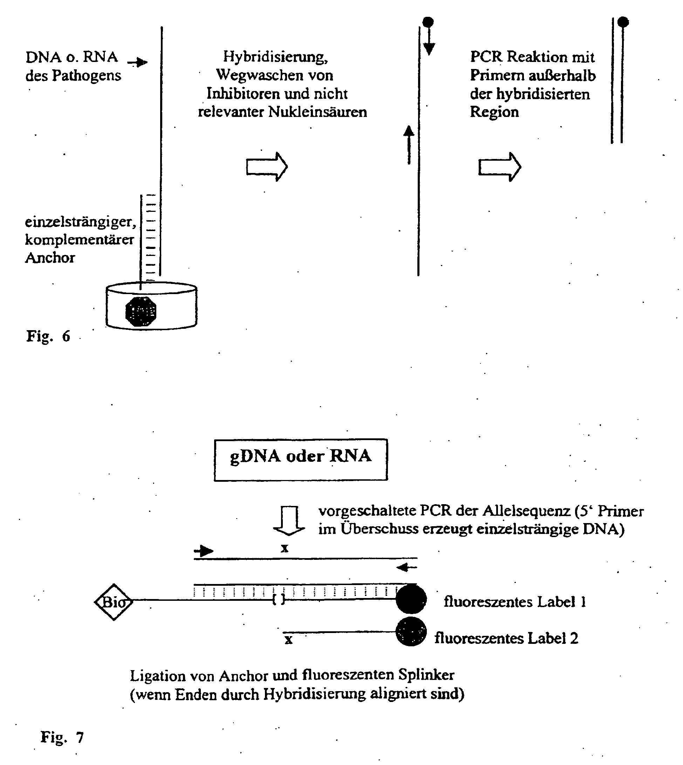

Figur 6 zeigt eine schematische Darstellung des Nachweises von Pathogenen nach Anreicherung durch PCR (PathoCheck™). Bio bedeutet eine Modifikation, mit der das Anchor-Oligonukleotid an eine feste Matrix gekoppelt ist.
Figur 7 zeigt eine schematische Darstellung der Identifizierung bekannter Allele durch Ligation markierter Splinker (LIMA™). Bio bedeutet eine Modifikation, mit der das Anchor-Oligonukleotid an eine feste Matrix gekoppeh ist. x bedeutet die Stelle, an der die zu bestimmende Modifikation vorliegt.

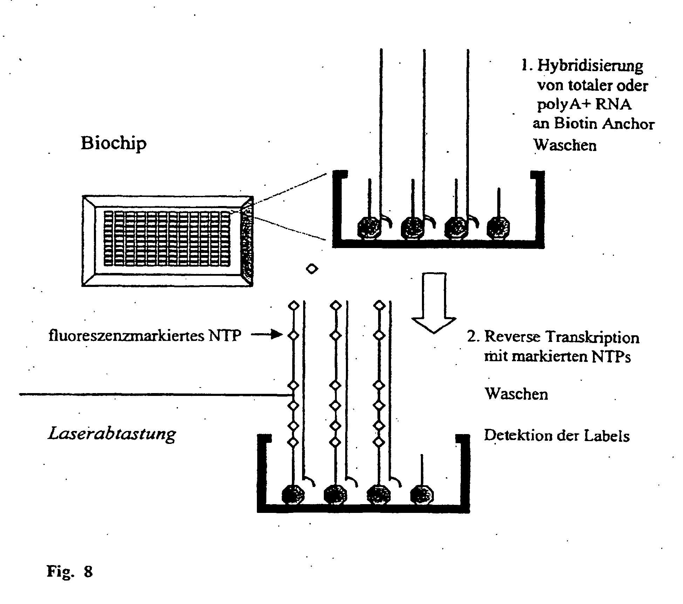

Figur 8 zeigt eine schematische Darstellung der Paralletanalyse von mRNA-Arrays (PAMINA™).

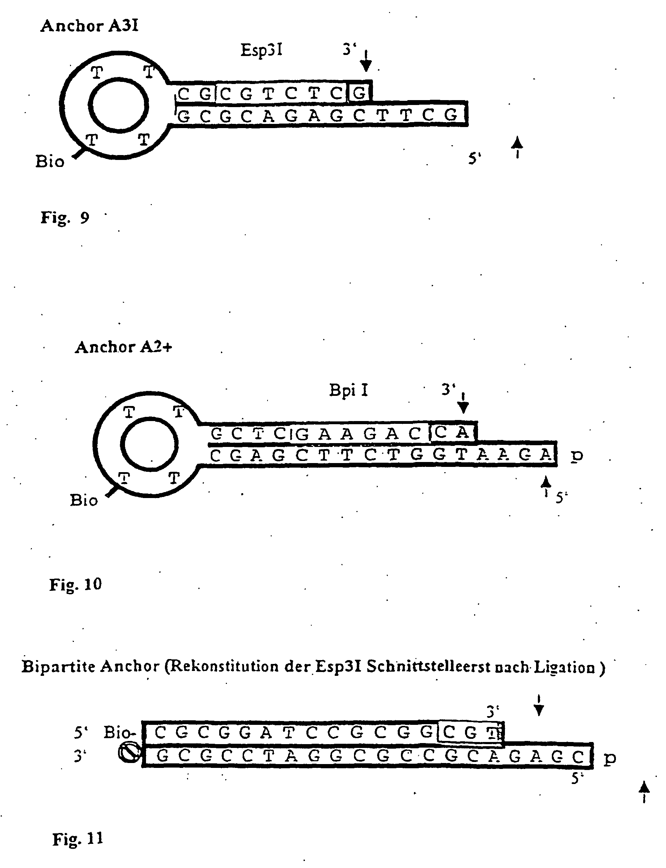

Figur 9 zeigt die schematische Darstellung eines Anchor-Oligonukleotids. Bio bedeutet eine Modifikation, mit der das Anchor-Oligonukleotid an eine feste Matrix gekoppelt ist. T, G, C, A bezeichnen die Nukleinsäurebasen, wobei T Thymidin, G Guanin, C Cytosin, A Adenin bedeutet. Esp3I bezeichnet ein Restriktionsenzym.
Figur 10 zeigt die schematische Darstellung eines Anchor-Oligonukleotids. Bio bedeutet eine Modifikation, mit der das Anchor-Oligonukleotid an eine feste Matrix gekoppelt ist. T, G, C, A bezeichnen die Nukleinsäurebasen, wobei T Thymidin, G Guanin, C Cytosin, A Adenin bedeutet. BpiI bezeichnet ein Restriktionsenzym.
Figur 11 zeigt die schematische Darstellung eines Bipartite-Anchor-Oligonukleotids. Bio bedeutet eine Modifikation, mit der das Anchor-Oligonukleotid an eine feste Matrix gekoppelt ist. T, G, C, A bezeichnen die Nukleinsäurebasen, wobei T Thymidin, G Guanin, C Cytosin, A Adenin bedeutet.

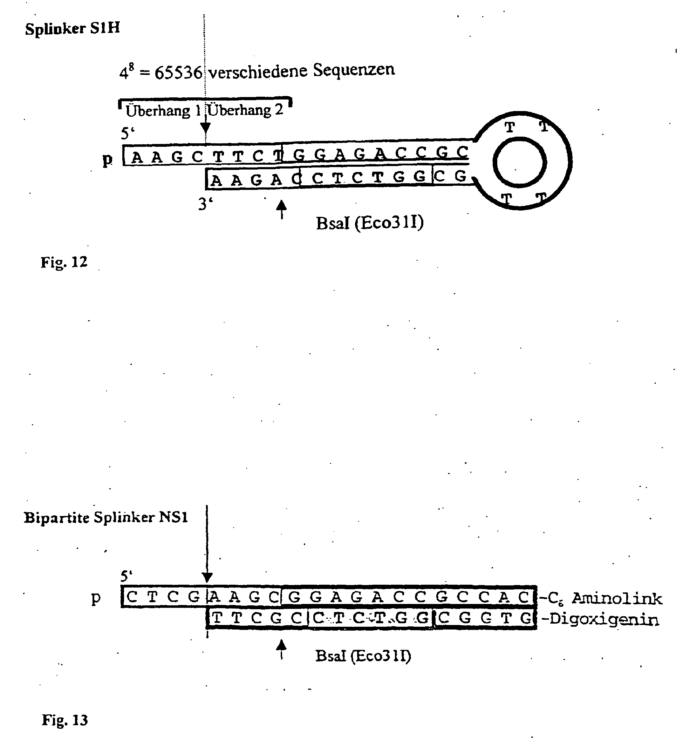

Figur 12 zeigt die schematische Darstellung eines Splinker-Oligonukleotids. T, G, C, A bezeichnen die Nukleinsäurebasen, wobei T Thymidin, G Guanin, C Cytosin, A Adenin bedeutet. BsaI und Eco31I bezeichnen Restriktionsenzyme.
Figur 13 zeigt die schematische Darstellung eines Blpartite-Splinker-Oligonukleotids. T, G, C, A bezeichnen die Nukleinsäurebasen, wobei T Thymidin, G Guanin, C Cytosin, A Adenin bedeutet. BsaI und Eco31I bezeichnen Restriktionsenzyme.

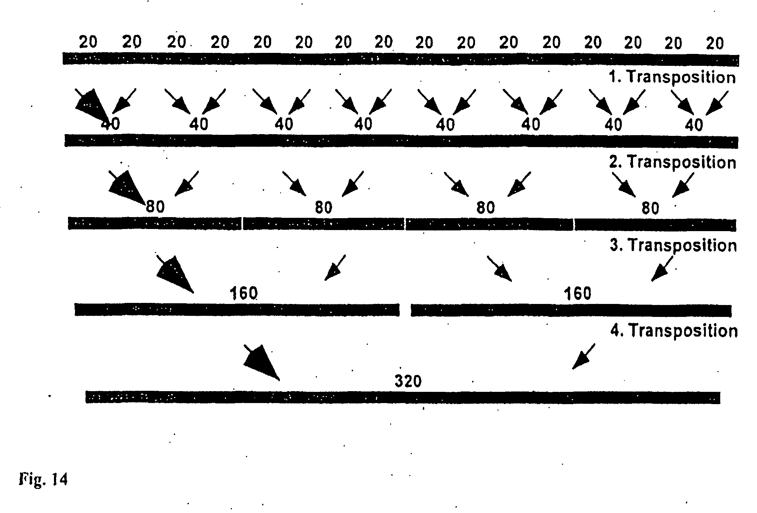

Figur 14 zeigt eine schematische Darstellung des Synthesewegs längerer Nukleinsäuren mit dem erfindungsgemäßen Verfahren. Die Balken symbolisieren doppelsträngige DNA-Fragmente, welche parallel durch hintereinander geschaltete Ligations/Restriktionszyklen synthetisiert wurden. Im Endprodukt benachbarte Teilabschnitte werden jeweils durch die Ligation eines aufligierten Splinkers mit einem aufligierten Anchor verbunden. Die so gewonnenen größeren Fragmente werden dann im nächsten Schritt wieder entweder mit der Anchor-spezifischen oder der Splinker-spezifischen Restriktionsendonuklease geschnitten und über komplementäre Überhänge miteinander verknüpft usw., so dass sich die Länge der Fragmente mit jedem Schritt verdoppelt. Die Verknüpfung ist vollkommen sequenzunabhängig, da die Erkennungssequenzen der verwendeten Restriktionsendonukleasen in den jeweils abgeschnittenen Teilen der aufligierten Fragmente liegen und daher nicht in die wachsende Nukleinsäure eingebaut werden. Die Zahlen über den Balken bedeuten die Größe der Fragmente in Basenpaaren. Ausgehend von 20 Basenpaar großen DNA-Fragmenten ergibt sich somit nach vier Transpositionen eine max. Länge von 320 Basenpaaren, nach fünf Transpositionen eine Länge von 640 Basenpaaren, nach sechs Transpositionen eine Länge von 1280 Basenpaaren, nach sieben Transpositionen eine Länge von 2560 Basenpaaren, usw.

### Definitionen

Der hier verwendete Begriff "parallel" oder "parallele Synthese" bedeutet, daß verschiedene erfindungsgemäße Nukleinsäuremolekule gleichzeitig in getrennten Reaktionsansätzen synthetisiert werden können, um dann mit dem erfindungsgemäßen Verfahren z.B. als Anchor oder Splinker zu einem verlängerten Nukleinsäuremolekule ligiert werden können.

Der hier verwendete Begriff "Sloning" (Sequentielle Ligation von Oligonukleotiden auf Sequenz-unabhängige Weise) bezieht sich auf ein Verfahren zur aufeinanderfolgenden Ligation von Oligonukleotiden mit beliebiger Sequenz.

Der hier verwendete Begriff "Anchor" oder "Anchor-Oligonukleotid" bezieht sich auf ein Oligonukleotid, welches über eine Modifikation an eine feste Matrix gekoppelt werden kann. Im Sinne der vorliegenden Erfindung enthält das Oligonukleotid in seinem doppelsträngigen Anteil ferner eine Restriktionsschnittstelle für ein TypIIS Restriktionsenzym, welches außerhalb seiner Erkennungssequenz schneidet.

Der hier verwendete Begriff "Splinker" oder "Splinker-Oligonukleotid" bezieht sich auf ein Oligonukleotid, welches über keine bzw. eine andersgeartete Modifikation verfügt, so daß es nicht selbst an die Matrix bindet, an welche die Anchor-Oligonukleotide gekoppelt sind.

Der hier verwendete Begriff "Dumbbell" (auf dt.: Glockenklöppel) bezieht sich auf eine DNA-Struktur, die durch einen Doppelstrang charakterisiert ist, der von zwei Loops flankiert wird.

Eines der beiden in jedem Reaktionsschritt zu verknüpfenden Oligonukleotide (das sog. "Anchor"-Oligonukleotid) ist über eine Modifikation, z.B. eine niedermolekulare chemische Verbindung wie Biotin oder Digoxigenin, an eine feste Matrix koppelbar. In einer bevorzugten Ausführungsform handelt es sich dabei um magnetische Streptavidin-beschichtete oder anti-Digoxigenin-beschichtete Beads. Das andere Oligonukleotid (das sog. "Splinker"-Oligonukleotid) besitzt auch ein bloekiertes Ende, aber keine derartige oder aber eine andersartige Modifikation. Der Punkt auf den es ankommt ist der, dass die Anchor-Oligonukleotide durch die Bindung an eine geeignete Matrix von den Splinker-Oligonukleotiden getrennt werden können. Es können daher beliebige Verbindungen z.B. Biotin, Digoxigenin, Fluoresceinisothiocyanat (FITC), Aminoverbindungen, Succinylester und andere dem Fachmann geläufige Verbindungen, verwendet werden, sofern sie geeignet sind, direkt oder indirekt (z.B. über einen Antikörper) eine Bindung an eine feste Phase zu vermitteln.

Anchor-Oligonukleotide können entweder aus einem einzigen zum Teil selbstkomplementären Oligonukleotid bestehen, welches über eine Modifikation vorzugsweise in der Loopsequenz an eine feste Phase koppelbar ist, oder aus zwei einzelsträngigen Oligonukleotiden, welche einen Doppelstrang bilden, vorzugsweise mit einem Einzelstrangüberhang. Da nur einer der beiden Stränge an eine Matrix gekoppelt werden muss, kann der andere wenn nötig durch Alkali oder Hitze denaturiert und abgetrennt werden (etwa um als Template für eine PCR-Reaktion zu dienen). Um sicherzugehen, daß auch bei solchen zweigeteilten Anchor-Oligonukleotiden nur ein Ende ligierbar ist, werden die nicht für die Ligation benötigten Enden entsprechend blockiert. Nukleinsäuresequenzen beispielhafter Anchor-Oligonukleotide sind

Splinker-Oligonukleotide können entweder aus einem einzigen zum Teil selbstkomplementären Oligonukleotid bestehen oder aus zwei einzelsträngigen Oligonukleotiden, welche einen Doppelstrang bilden, vorzugsweise mit einem Einzelstrangüberhang, d.h. man hat ein mindestens teilweise komplementäres Paar von Oligonukleotiden, wobei die jeweils nicht zu ligierenden Enden der beiden Einzelstränge blockiert sein müssen. Die bevorzugte Einzelstrangüberhangsequenz muss zu dem jeweils zu ligierenden Anchor-Oligonukleotid komplementär sein. Nukleinsäuresequenzen beispielhafter Splinker-Oligonukleotide sind

Sowohl Anchor- wie auch Splinker-Oligonukleotide können Überhänge einer definierten Länge enthalten, in einer bevorzugten Ausführungsform eins bis fünf Nukleotide. Diese Überhänge sind bei den jeweils zu ligierenden Oligonukleotiden komplementär zueinander, am 5' Ende phosphoryliert und können nur in einer Orientierung miteinander ligiert werden. Dabei entsteht ein ligiertes Oligonukleotid mit z.B. einer sogenannten "Dumbbell"-Struktur. Um eine vollständige Ligation aller verfügbaren Anchor Oligonukleotide zu erreichen, können die anzuligierenden Splinker-Oligonukleotide in 2 bis 10fachem Überschuß zugesetzt werden. Die überschüssigen, nicht-reagierten Splinker werden nach jedem Ligationsschritt mit Puffer weggewaschen. Werden z.B. mit Streptavidin beschichtete magnetische Beads verwendet, können die Beads mit den über eine Streptavidin/Biotin-Bindung gebundenen Anchor-Oligonukleotiden zusammen mit den anligierten Splinkern durch den Einsatz eines Magneten im Reaktionsansatz zurückgehalten werden. Alternativ können z.B. direkt mit Streptavidin beschichtete Wells, Glas(beads), Objektträger, DNA-Chips oder beliebige andere feste Phasen verwendet werden. Beads werden in der Regel bevorzugt, weil sie eine größere Oberfläche und daher eine höhere Bindungskapazität aufweisen.

Um weitere Ligationen durchführen zu können, muss eine Erkennungssequenz für eine Restriktionsendonuklease vorhanden sein, die die Nukleinsäuresequenz außerhalb dieser Erkennungssequenz in dem anligierten Splinker-Oligonukleotid schneidet. Beispiele für solche Enzyme sind BpiI, Esp3I, Eco31I, SapI etc. Für das erfindungsgemäße Verfahren nützliche Restriktionsenzyme und ihre Erkennungssequenzen und Schnittstellen sind in der Rebase-Datenbank unter http://rebase.neb.com/rebase/rebase.html zu finden. An der in den Splinker-Oligonukleotiden enthaltenen Restriktionsschnittstelle werden die Ligationsprodukte so geschnitten, daß ein Teil der Splinkersequenz am Anchor-Oligonukleotid verbleibt. Gleichzeitig wird dadurch ein Sequenzüberhang erzeugt, der für die Ligation eines weiteren Splinker-Oligonukleotids herangezogen werden kann. Der andere abgespaltene Teil und der nicht ligierten Rests des Splinker-Oligonukleotids, das Restriktionsenzym sowie der Restriktionspuffer werden aus dem Reaktionsansatz ausgewaschen, worauf ein weiterer Zyklus beginnt. Der Zyklus kann entweder nur einmal durchgeführt oder aber mehrere Male wiederholt werden, bevor die so verlängerten Oligonukleotide ihrerseits mit den parallel synthetisierten Nachbarfragmenten verknüpft werden. Da die beim Schneiden mit den verschiedenen Restriktionsendonukleasen entstehenden, jeweils zueinander komplementären Überhänge aus dem zu synthetisierenden Gen stammen, die Erkennungssequenzen hingegen in den wieder abgeschnittenen Teilen der Anchor- bzw. Splinker-Oligonukleotide lokalisiert sind, können die benachbarten Fragmente völlig unabhängig von ihrer Sequenz verknüpft werden. Insbesondere lassen sich dadurch selbst große Gene in vielen parallel ablaufenden Teilreaktionen in nur wenigen Reaktionsschritten zusammen bauen. Im optimalen Fall lässt sich beispielsweise ein 2 kb großes Gen in lediglich 9 Schritten aus 256 Einzelreaktionen zusammen setzen. Ein gleich großes Gen würde bei linearer Synthese (rekursiv, aber nicht parallel) und bei Verwendung von 60mer Oligos mehr als 30 Schritte benötigen. Da sowohl enzymatische als auch chemische Ligationsverfahren Ausbeuten von meist nur 80-90% aufweisen, nimmt die Gesamtausbeute mit der Anzahl der benötigten Reaktionsschritte exponentiell ab, weswegen Verfahren mit wenigen Reaktionsschritten vorteilhaft sind. Um nicht-umgesetzte Anchor-Oligonukleotide von der weiteren Synthese auszuschließen, kann fakultativ nach der Ligation ein Exonuklease- und/oder Phosphatase-Schritt zwischengeschaltet werden, wodurch der Überhang oder zumindest die für die folgende Ligation erforderliche 5'-Phosphatgruppe entfernt wird. Der Anteil an nicht-umgesetzten Anchor-Oligonukleotiden ist bei einem eingesetzten Überschuß an Splinker-Oligonukleotiden nur gering. Eine Weiterreaktion sollte zudem nur dann möglich sein, wenn dieselbe Sequenz ein weiteres Mal anligiert wird, weswegen die Gefahr einer Kontamination mit nicht oder nur teilweise umgesetzten Anchor Oligonukleotiden als relativ gering anzusehen ist.

Die so nach mehreren Ligations- und Restriktionszyklen aufligierte Nukleinsäuresequenz kann anschließend durch Schneiden mit einem Restriktionsenzym, das eine Nukleinsäuresequenz in dem ursprünglichen Anchor-Oligonukleotid spezifisch erkennt, von dem an der Matrix verbleibenden Anchor-Oligonukleotid abgetrennt werden. Die aufligierte Nukleinsäuresequenz hängt nun an dem zuletzt anligierten Splinker-Oligonukleotid. Das verlängerte Splinker-Oligonukleotid wird nach Inaktivierung des Restriktionsenzyms aus dem ursprünglichen Reaktionsansatz in ein neues Reaktionsgefäß überführt und dort mit einem aufligierten Anchor-Oligonukleotid verknüpft, das mit einem Splinker-Oligonukleotid spezifischen Restriktionsenzym geschnitten wurde (1. Transposition). Dem Fachmann ist ersichtlich, daß es sich bei den aufligierten Nukleinsäuresequenzen um frei wählbare Sequenzen handeln kann, die sowohl unterschiedlich als auch identisch sein können. Das aus der 1. Transposition resultierende Ligationsprodukt wird wiederum mit einer Anchor-spezifischen Restriktionsendonuklease geschnitten und erneut mit einem analog erhaltenen aufligierten Anchor-Oligonukleotid ligiert (2. Transposition). Auf diese Weise verdoppelt sich die Länge der aufligierten Nukleinsäuresequenzen dann mit jedem weiteren Schritt. Die Verknüpfung der DNA-Fragmente erfolgt jeweils über komplementäre Überhänge, ist aber ansonsten vollkommen sequenzunabhängig. Die einzige Einschränkung dabei ist, daß die Anchor- und Splinker-spezifischen Restriktionsschnittstellen in der zu synthetisierenden Sequenz nicht vorkommen dürfen, weil sonst die DNA auch intern geschnitten werden würde. Jeweils vor einer Spaltung an einer Anchor-spezifischen Restriktionsschnittstelle und der darauf folgenden Transposition kann fakultativ ein Exonukleaseschritt eingeführt werden, um die Transposition nicht vollständig aüfligierter Splinker-Oligonukleotide zu verhindern. Die für das Verfahren notwendigen sequenzspezifischen Spaltungen können statt durch TypIIS Restriktionsendonukleasen im Prinzip auch durch analog funktionierende Ribozyme erfolgen.

Ausgehend von einer 20 Basenpaar langen Sequenz (die man für Splinker mit einem 4 nt Überhang durch 5 sukzessive Ligationen der hierfür benötigten Ursprungs-Splinker aus der Bibliothek erhalten kann), lässt sich durch lediglich 7 weitere Ligationsschritte eine doppelsträngige DNA-Sequenz von 2560 Basenpaaren Länge synthetisieren. Bei Zykluszeiten von ca. 1 Stunde kann eine beliebige DNA-Sequenz dieser Länge binnen 12 Stunden synthetisiert werden. Durch eine Optimierung der Reaktionsbedingungen kann der Zeitaufwand auf etwa 6 Stunden halbiert werden.

Bei 4 Nukleotide langen Überhängen wird eine Bibliothek von 65536 verschiedenen Splinker-Oligunukleotiden benötigt, um alle möglichen Nukleinsäuresequenzen herstellen zu können. Diese Zahl ergibt sich aus folgender Berechnung: es gibt 256 mögliche 4 Nukleotide lange Überhänge (4⁴ = 256), ebenso viele Sequenzvarianten existieren für die vier direkt angrenzenden Nukleotide, die den Überhang beim nächsten Ligationsschritt bilden. Insgesamt ergibt sich daraus eine Gesamtzahl von 4⁴ mal 4⁴ = 4⁸ = 65536 Splinker-Oligonukleotide, mit denen sämtliche möglichen Sequenzvarianten dargestellt werden können. Bei 3 Nukleotide langen Überhängen reduziert sich die Komplexität der benötigten Splinkerbibliothek entsprechend auf 4³ mal 4³ = 4096, bei zwei Nukleotide langen Überhängen auf 4² mal 4² = 256, bei 5 Nukleotide langen Überhängen würde sie sich auf 4⁵ mal 4⁵ = 1048576 erhöhen. Voraussetzung für dieses Baukastensystem ist das Vorhandensein einer kompletten Splinkerbibliothek (für 2 nt Überhänge 256 Oligonukleotide, für 3 nt Überhänge 4096 Oligonukleotide, für 4 nt Überhänge 65536 Oligonukleotide, für 5 nt Überhänge 1048576 Oligonukleotide) sowie einer Anchorbibliothek (4, 16, 64, 256 oder 1024 Oligonukleotide bei 1, 2, 3, 4 oder 5 nt Überhängen). Letztere ist allerdings nicht unbedingt nötig, da die verschiedenen Überhangsequenzen ebenso gut durch einen vorgeschalteten Ligationsschritt mit geeigneten Splinker-Oligonukleotiden erzeugt werden können.

Prinzipiell sind alle Einzelschritte des erfindungsgemäßen Verfahrens automatisierbar, so daß die Herstellung ganzer Gene so einfach ist wie die Synthese von Oligonukleotiden. Zudem eröffnet sich durch das erfindungsgemäße Verfahren ein Kostensenkungspotential in beträchtlicher Höhe. Erstens können alle benötigten Enzyme großtechnisch hergestellt werden. Zweitens können die Investitionen für die Splinkerlibrary deutlich gesenkt werden, indem die einzelnen Splinker-Oligonukleotide bis auf die letzten 4 Nukleotide des 5'-Überhangs *en bloc* synthetisiert werden. Die Synthesereaktion wird dann in 4 gleiche Teile portioniert; die vier verschiedenen Nukleotide werden dann in separaten Reaktionen an der nächsten (im Endprodukt viertletzten) Position angehängt. Danach werden die vier Einzelreaktionen wiederum geviertelt, wonach das drittletzte Nukleotid angehängt wird usw. Statt 65536 Einzelsynthesen würde man dann nur 256 Synthesen in einem entsprechend größeren und deswegen günstigeren Maßstab benötigen. Ferner können die 256 möglichen 4 Nukleotide langen Überhänge durch eine "blunt end ligation" an 256 verschiedene Anchor-Oligonukleotide, anschließender Exonukleasebehandlung, Waschen und schließlich Restriktion mit der ***Anchor***-spezifischen Restriktionsendonuklease erzeugt werden. Auf diese Weise könnten die 65536 benötigten Splinker-Oligonukleotide kostengünstig hergestellt werden. Ferner könnte auf diese Art und Weise eine aufwendige Aufreinigung aller 65536 Splinker-Oligonukleotide umgangen werden, da nichtreaktive Fehlsequenzen durch dieses Verfahren entfernt werden. Da eine extrem hohe Reinheit der eingesetzten Oligonukleotide essentiell für das Gelingen fehlerloser Synthesen ist, müssen diese sowieso entsprechend vorbehandelt werden. Daneben muss eine praktisch vollständige Abwesenheit von Exonukleasen während der Restriktions- und Ligationsschritte gewährleistet sein, damit die Überhangsequenzen intakt bleiben, die für die nachfolgenden Ligationen benötigt werden. Vor allem wenn Exonuklease-Zwischenschritte zur Entfernung nichtligierter Anchor-Oligonukleotide verwendet werden, müssen diese Exonukleasen gründlich weggewaschen und/oder inaktiviert werden.

Die Anchor- und Splinker-Oligonukleotide können sowohl jeweils aus einem selbst-komplementären Einzelstrang als auch aus je zwei komplementären Plus- und Minus-Strängen zusammengesetzt werden. Die Nukleinsäuresequenzen müssen nicht vollständig komplementär sein; die selbstkomplementären Einzelstrang-Oligonukleotide können einen Loop aufweisen und die komplementären Plus- und Minus-Stränge können nur teilweise komplementär sein. Bei Anchor- und Splinker-Oligonukleotiden, die aus je zwei komplementären Plus- und Minus-Strängen zusammengesetzt sind, muss (i) die Schmelztemperatur des Doppelstranghybrids genügend hoch sein, um eine Denaturierung der zusammengesetzten Anchor- und Splinker-Oligonukleotide und einen möglicherweise daraus resultierenden unbeabsichtigten Transfer der nicht an eine Festphase gekoppelten Einzeistränge zu verhindern und müssen (ii) die jeweils nicht zu verlängernden Enden durch geeignete Modifikationen blockiert sein. Oligonukleotide aus zwei komplementären Plus- und Minus-Strängen besitzen gegenüber Oligonukleotiden aus einem selbstkomplementären Einzelstrang bestimmte Vorteile. Selbstkomplementäre (Snap back) Oligonukleotide verursachen bei der Aufreinigung oft gewisse Schwierigkeiten, da sie in hoher Konzentration eine Tendenz zur Bildung von Netzwerken haben. Einzelsträngige Teiloligonukleotide sind auch kürzer und dadurch mit geringerem Aufwand in höherer Reinheit zu gewinnen. Für bestimmte erfindungsgemäße Ausführungsformen werden aus zwei Teiloligonukleotiden zusammengesetzte ("bi-partite") Anchor-Oligonukleotide verwendet.

In besonders bevorzugten Ausführungsformen enthalten die Anchor bzw. Splinker die folgenden Kombinationen an Erkennungssequenzen:

| Anchor | Splinker |
|---|---|
| CGTCTCN^NNNN_(Esp31, BsmBI) | GGTCTCN^NNNN_(BsaI, Eco31L,..) |
| GGTCTCN^NNNN_(BsaI, Eco31L,..) | CGTCTCN^NNNN_(Esp3I, BsmBI) |
| GAAGACNN^NNNN_(BbsI, BpiI...) | ACCTGCNNNN^NNNN_(BspMI, Acc36I) |
| ACCTGCTNNNN^NNNN_(BspMI, Acc36I) | GAAGACNN^NNNN_(BbsI, BpiI...) |
| GCAGTG_NN^ (BtsI) | GCAATG_NN^(BsrDI, Bse3DI, ..) |
| GCAATG_NN^ (BsrDI, Bse3DI, ..) | GCAGTG_NN^ (BtsI) |
| GTATCCNNNNN_N^ (BciVI, BfuI) | ACTGGGNNNN_N^ (BfiI, BmrI) |
| ACTGGGNNNN_N^ (BfiI, BmrI) | GTATCCNNNNN_N^ (BciVI, BfuI) |

Das erfindungsgemäße Kit kann neben den Merkmalen der entsprechenden unabhängigen Anspruches aus magnetisierten Beads, geeigneten Reaktionsbehältnissen, Ligase, gegebenenfalls einer Topoisomerase und/oder einer 3'-5'-Exonuklease und/oder Phosphatase, sowie allen benötigten Reaktionspuffer bestehen. Bevorzugt ist fernerhin eine Pipettierstation mit einem kühlbaren Probenvorratsbehälter mit einer entsprechenden Softwaresteuerung, die sämtliche Schritte des erfindungsgemäßen Verfahrens automatisch durchführt

Die vorliegende Erfindung erlaubt eine komplette Automatisierung des gesamten Gensyntheseprozesses durch die Bereitstellung von einer Bibliothek immer wieder zu benützender, mindestens teilweise doppelsträngiger hochreiner Oligonukleotide mit Erkennungssequenzen für bestimmte TypIIS Restriktionsendonukleasen (sogenannte "Outside Cutter"). Die Automatisierung wird ferner erlaubt durch die Bereitstellung eines Verfahrens, welche die parallele Synthese von Genfragmenten und deren sequenzunabhängige Verknüpfung an jeder beliebigen Stelle erlaubt sowie aufgrund einer orientierungsgebundenen Verlängerung der Startmoleküle über deren Bindung an eine Festphase (die nicht zu ligierenden Enden sind durch geeignete Modifikationen bzw. Loopsequenzen blockiert) und eines definierten Sets rekursiver Prozeduren (Ligations-, Wasch- und Restriktionsschritte), die durch einen Roboter abgearbeitet werden können.

Nachstehend werden bestimmte Aspekte der vorliegenden Erfindung beispielhaft wiedergegeben, die auf der Komplettsynthese ganzer Gene durch das erfindungsgemäße Verfahren beruhen.

### 1. Herstellung einer cDNA, wenn nur die Proteinsequenz bekannt ist

Es kommt häufig vor, daß nur die Aminosäuresequenz oder Teile der Aminosäuresequenz eines Proteins bekannt sind, jedoch nicht die cDNA oder genomische Sequenz. Wegen der Degeneration des genetischen Codes ist es in der Regel nicht möglich, das entsprechende Gen direkt über eine PCR einer geeigneten cDNA-Bank zu amplifizieren. Man sucht daher Regionen, in denen Aminosäuren wie Tryptophan, Methionin bzw. Asparagin, Aspartat, Glutamat, Glutamin, Tyrosin, Phenylalanin, Cystein oder Lysin gehäuft auftreten, da es für diese Aminosäuren nur ein bzw. zwei Codons gibt. Sofern es gelingt, mit niedrig degenerierten Primern ein PCR-Fragment der erwarteten Größe zu erhalten, wird dieses als Sonde benutzt, um das dazugehörige Gen aus einer cDNA-Bank zu klonieren. Diese Arbeit wird zwar heutzutage durch die Verfügbarkeit von Gene-Arrays und Klonkollektionen in vielen Fällen wesentlich erleichtert, doch zum einen stehen solche Hilfsmittel nur für eine begrenzte Anzahl von Organismen und Zelltypen zur Verfügung, zum anderen ist selbst bei Vorliegen der kompletten cDNA meist noch eine Umklonierung in einen geeigneten Expressionsvektor erforderlich. Der Zeitaufwand kann je nach Schwierigkeit des Projekts bei ein bis zwei Wochen, in Extremfällen aber durchaus auch bei mehreren Monaten bis Jahren liegen. Mit dem erfindungsgemäßen Verfahren kann ausgehend von einer bekannten Proteinsequenz ein Expressionskonstrukt mit einem für den gewünschten Organismus optimierten Codon Usage in ein bis zwei Tagen hergestellt werden. Hierfür muss der Organismus, in dem das Protein natürlicherweise exprimiert wird, gar nicht zur Verfügung stehen, da die DNA-Sequenz aus der bekannten Proteinsequenz abgeleitet werden kann, ohne daß ein Template vorliegen muss. Mit verbesserten Proteinsequenzierungsmethoden wird es in Zukunft möglich sein, Proteine mit interessanten Enzymaktivitäten aus beliebigen Organismen zu sequenzieren und ohne den Umweg über die cDNA-Klonierung mittels des erfindungsgemäßen Verfahrens direkt in jedes gewünschte Expressionssystem zu überführen.

### 2. Herstellung von Designergenen und Designerproteinen

Eine weiterer Aspekt der vorliegenden Erfindung ist die einfache Herstellung von Designergenen bzw. Designerproteinen, d.h. die Kopplung funktioneller Domänen verschiedener Proteine, um beispielsweise Enzyme mit neuen oder veränderten Eigenschaften herzustellen. Bei Kenntnis der Röntgenkristallstruktur eines Proteins können dann sehr gezielte Veränderungen wie z.B. die Einfügung definierter Linkerdomänen oder ein Redesign einer Bindungstasche vorgenommen werden, um neue Funktionen oder veränderte Spezifitäten in Proteine einzuführen. Durch zielgerichtetes Proteindesign kann man beispielsweise regulierbare katalytische Zentren konstruieren, die durch eine Konformationsänderung des Proteins infolge der Bindung eines spezifischen Liganden aktiviert werden. Auf diese Weise können Designerproteine hergestellt werden, die z.B. auf die Bindung eines bestimmten Virusproteins hin eine Caspase-Aktivität entfalten, die dann in infizierten Zellen Apoptose auslöst. Erste Versionen solcher hochspezifischen Pharmaka sind bereits beschrieben worden; vgl. Vocero-Akbani A.M., Heyden N.V., Lissy N.A., Ramer L., Dowdy S.F., Nat Med, 1999 Jan, 5:1, 29-33. Weiterhin können Proteine dadurch stabilisiert werden, daß an bestimmten Positionen Aminosäuren eingebaut werden, die zusätzliche Salzbrücken bilden können. Somit kann die Toleranz gegenüber hohen Temperaturen verbessert werden, was unter anderem für die Waschmittelindustrie vorteilhaft ist. Sofern die Domänstrukturen bekannt sind, kann durch die punktgenaue Expression bestimmter funktioneller Regionen eine gewünschte enzymatische Aktivität von einer unerwünschten getrennt werden. Ebenso lassen sich Multienzymkomplexe konstruieren, die eine ganze Reihe verschiedener Reaktionen katalysieren können. Dadurch lassen sich Verbesserungen bei der Synthese vieler organischer Verbindungen erreichen oder manche Synthesen sogar erst ermöglichen. Dies eröffnet ganz neue Perspektiven, da viele organische Synthesen, bei denen heute noch umweltgefährdende Lösungsmittel und Katalysatoren eingesetzt werden müssen, in Zukunft durch solche maßgeschneiderten Biokatalysatoren ersetzt werden können.

### 3. Systematische Mutagenese als Ersatz für randomisierte Mutagenisierung

Eine häufig vorkommende Aufgabe in der biochemisch orientierten Molekularbiologie besteht darin, aus vielen Proteinvarianten diejenige herauszusuchen, die die höchste enzymatische Aktivität oder die stärkste Bindung an ein Substrat oder ein anderes Protein aufweist. Man geht dann meist so vor, daß man eine Reihe von zufälligen Mutationen von einer oder mehreren Aminosäuren einführt und die entstehenden Varianten in einem geeigneten Screening-Verfahren analysiert. Zwar ist es prinzipiell auch möglich, alle Mutanten separat herzustellen, jedoch wird dies aus Zeit- und Kostengründen selten durchgeführt. Bei einer randomisierten Mutagenisierung ist die Kontrolle über die entstehenden Mutanten naturgemäß sehr limitiert, zum einen da bestimmte Aminosäuresubstitutionen verfahrensbedingt häufiger gefunden werden als andere, zum anderen, da es sich kaum vermeiden lässt, daß bei diesem Verfahren auch Stopcodons eingeführt werden. Mit dem erfindungsgemäßen Verfahren lassen sich hingegen alle gewünschten Mutanten gezielt und ohne großen Aufwand darstellen und als Proteine exprimieren.

### 4. Herstellung synthetischer Gene, insbesondere zum Einsatz als DNA-Vakzine

In vielen Fällen ist es wünschenswert, die Proteinexpression bestimmter Gene in heterologen Systemen zu optimieren. Dies kann durch die Verwendung starker Promotoren sehr häufig nur zum Teil erreicht werden. Je nachdem, welcher Organismus für die Expression verwendet wird, kann sich die Verwendung bestimmter Codons für eine Aminosäure vorteilhaft oder nachteilhaft auf die erreichbare Genexpression auswirken. So sind beispielsweise viele retrovirale Genprodukte in eukaryotischen Zellen nur schlecht translatierbar, da sie meist sehr AT-reich sind und in höheren Eukaryonten seltene Codons benutzen. Speziell für den Einsatz solcher Gensequenzen als DNA-Vakzine ist es daher von großem Vorteil, wenn deren Codongebrauch für Säugerzellen optimiert ist. Desgleichen können bestimmte RNA-Strukturen zu einer Instabilität der Transkripte führen, was die Genexpression ebenfalls negativ beeinflussen kann. Solche Elemente können durch Codonveränderungen mit dem erfindungsgemäßen Verfahren ebenfalls leicht ausgeschaltet werden.

### 5. Analyse von Proteindomänen durch Deletions- oder Punktmutagenese

Die Analyse von Mutanten ist sehr oft das Mittel der Wahl bei der funktionellen Charakterisierung von Proteinen. Zwar existiert sowohl für die Herstellung von Deletions- wie auch Punktmutanten eine Reihe etablierter Verfahren, jedoch sind diese in der Regel sehr zeitaufwendig und arbeitsintensiv. Deletionen werden meist durch Einführen von Linkersequenzen oder durch eine PCR mit Primern, deren Enden zu verschiedenen Teilsequenzen komplementär sind, hergestellt. Um eine ganze Serie definierter Deletionen zu erhalten, ist häufig ein Zweischrittverfahren notwendig, bei dem zunächst bestimmte Restriktionsschnittstellen eingeführt werden, über welche dann die gewünschten Deletionen eingeführt werden können. Mit entsprechend konzipierten Primern und einer Mehrfragmentligation lassen sich solche Deletionen zwar im Prinzip auch in einem Schritt herstellen, jedoch sind die Erfolgsaussichten dabei eher gering. In allen diesen Fällen muss die Wildtyp-DNA als Template vorliegen, was bei dem erfindungsgemäßen Verfahren nicht notwendig ist. Darüber hinaus können Deletionsmutanten hergestellt werden, da es gar nicht notwendig ist, Restriktionsschnittstellen einzuführen, für die man erst geeignete Stellen finden muss, damit die eingeführten Mutationen keine Veränderungen in der Proteinsequenz bewirken (sogenannte "Silent site" Mutationen). Auch die Herstellung von Doppel- oder Tripletmutanten ist mit dem erfindungsgemäßen Verfahren möglich. Für die funktionelle Kartierung eines Proteins können in dessen Gensequenz mittels der erwähnten "Silent site" Mutationen gleichzeitig auch Restriktionsschnittstellen für eine große Anzahl verschiedener Restriktionsendonukleasen eingeführt werden, mit deren Hilfe beliebige Deletionen hergestellt werden können. In vielen Fällen wird daher die klassische Mutationsanalyse verzichtbar und kann durch das schnellere und genauere erfindungsgemäße Verfahren ersetzt werden.

### 6. Gekoppelte in vitro Transkriptions/Translationssysteme ("EasyPro™")

Gekoppelte in vitro Transkriptions/Translationssysteme werden zur schnellen Synthese von Proteinen im analytischen Maßstab eingesetzt, z.B. für Bindungsstudien oder Kopräzipitationsassays. Hierfür werden die zu exprimierenden Sequenzen in einen Vektor kloniert, der einen Promoter für eine RNA-Polymerase enthält. Mit Hilfe dieser Polymerase wird mRNA transkribiert, die in einem RNA-depletierten Weizenkeim- oder Retikulozytenextrakt in das gewünschte Protein translatiert wird, das aufgrund der geringen Ausbeute und der einfacheren Nachweisbarkeit meist mit ³⁵S-Methionin oder Cystein radioaktiv markiert ist. Eine noch schnellere Alternative ist das auf dem erfindungsgemäßen Verfahren basierende EasyPro™-System. In einem Anchor-Oligonukleotid, welches einen T7 (SP6) Promoter, eine interne Ribosomenbindungsstelle sowie ein Hexahistidin-Tag enthält, wird durch Restriktion mit XcmI ein einzelner Thytnidinüberhang erzeugt, der direkt mit einem PCR-Produkt ligiert werden kann. Drei EasyPro™-Anchor-Oligonukleotide mit verschiedenen Leserastern sind ausreichend, um alle in der richtigen Orientierung ligierten PCR-Fragrnente zu translatieren. Mittels terminaler Transferase oder durch Ligation eines entsprechenden Splinker-Oligonukleotids an das 3'-Ende des PCR-Pmdukts kann man zudem leicht einen künstlichen poly-A-Schwanz in das DNA-Template einführen, welcher das RNA-Transkript stabilisiert und dadurch für eine höhere Translationseffizienz sorgt. Ferner können die für das gewünschte Protein codierenden DNA-Sequenzen nach Spaltung mit einer Restriktionsendonuklease auch direkt an einen modifizierten EasyPro™-Anchor mit einem passenden 4 nt langen Überhang ligiert werden.

In einer Ausführungsform wird das erfindungsgemäße Verfahren zur schnellen Synthese von Proteinen in einem Minireaktor verwendet. In der unteren Reaktionskammer des Minireaktors findet die Transkription der an Streptavidin-beschichtete Beads gekoppelten Expressions-Anchor-Nukleinsäuresequenz statt. Die dabei entstehenden mRNAs werden über ihren 3'-poly-A-Schwanz an Oligo-dT gekoppelte Beads gebunden, welche sich ebenfalls in der unteren Reaktionskammer befinden. Dort läuft auch die Translation der mRNAs in einem Retikulozytenextrakt ab. Diese Kammer wird durch eine Ultrafiltrationsmembran mit einem MWCO (Molekulargewichtsausschluss) von ca. 200 kD von einer darüber liegenden zweiten Kammer abgetrennt. Diese enthält Beads, welche das Protein von Interesse binden können (z.B. Ni²⁺-NTA-Beads für Proteine mit einem Hexahistidin-Tag). Durch eine kontinuierliche Zufuhr von Pufferlösung mit frischen niedermolekularen Reaktanden (Aminoacyl-tRNAs, Ribonukleotidtriphosphate, CAP-Analogon und Creatinphosphat) wird die Produktion über längere Zeit hinweg aufrechterhalten. Gleichzeitig wird dadurch das synthetisierte Protein aus der unteren in die obere Reaktionskammer gedrückt, wo es an den Beads hängen bleibt. Alternativ kann diese Kammer durch eine weitere Ultrafiltrationsmembran abgeschlossen werden, deren Ausschluß so gewählt ist, daß sie für Puffer und kleinere Moleküle, nicht aber für das gewünschte Protein permeabel ist. Dieses sammelt sich daher in der oberen Kammer an und kann von dort in aufgereinigter Form isoliert werden. Die dabei erzielbaren Ausbeuten sind nicht nur für die meisten analytischen Experimente ausreichend, sondern können sogar Proteinexpressionsexperimente im kleinen Maßstab ersetzen. Wenn es beispielsweise darum geht, die spezifische enzymatische Aktivität verschiedener Proteinmutanten zu bestimmen, mussten diese hierfür bislang in aufwendigen Vorversuchen kloniert, exprimiert und aufgereinigt werden. Da praktisch alle diese Schritte in dem erfindungsgemäßen EasyPro™-Verfahren bereits integriert sind, wird dadurch ein erheblicher Zeitvorteil gegenüber konventiellen Methoden erreicht.

Mit einer Modifikation des vorstehend beschriebenen erfindungsgemäßen Verfahrens können einfach und kostengünstig Peptidbibliotheken hergestellt werden, welche unter anderem zum Epitopmapping von Antikörpern oder zur Identifizierung immunogener Epitope in Proteinen von Viren, Bakterien oder Pilzen benötigt werden (zur schnellen Etablierung serologischer Nachweissysteme). Hierfür werden modifizierte EasyPro™-Anchor-Oligonukleotide sukzessive durch Splinkerligationen verlängert, so daß die für die gewünschten Peptide codierenden Sequenzen entstehen. Im letzten Schritt wird ein vorgefertigter Endsplinker anligiert, welcher für ein C-terminales Tag, ein Stopcodon sowie einen Poly-A-Schwanz codiert. Die Ligationsprodukte werden in dem beschriebenen Minireaktor transkribiert und translatiert. Die fertigen Peptide werden nach Abschluss der Translation und mehreren Waschschritten mit einer spezifischen Protease, z.B. Enterokinase oder Faktor Xa, an der vom EasyPro™-Anchor-Oligonukleotid codierten Schnittstelle abgespalten und aus der oberen Reaktionskammer ausgewaschen. Mit Hilfe des C-terminalen Tags können diese an eine feste Phase für nachfolgende Tests gebunden werden. Die Peptide liegen zudem schon in aufgereinigter Form vor und können direkt für nachfolgende Applikationen eingesetzt werden. Da jeweils das gleiche Anchor-Oligonukleotid verwendet wird und die benötigten Splinker-Oligonukleotide aus einem bereits vorgefertigten Teilstück in wenigen Schritten aufligiert werden können, sind die anfallenden Kosten geringer als bei einer konventionellen Peptidsynthese.

### 7. Herstellung von Ribozymen oder Aptameren

Analog zu der oben beschriebenen Proteinsynthese lassen sich Anchor-Oligonukleotide mit einem T7 (SP6) Promoter auch zur Herstellung und Mutagenisierung von RNAs verwenden. Das System eignet sich insbesondere zur Synthese verschiedener Ribozyme, da die für die Ribozyme codierenden DNA-Sequenzen auf einem verlängerten Splinker-Oligonukleotid an ein Promotormodul auf einem Anchor-Oligonukleotid anligiert werden können. Vor allem können genau definierte Ribozymtemplatebibliotheken erstellt werden, die sich per PCR leicht amplifizieren lassen. Mit dem erfindungsgemäßen Verfahren können Ribozymtemplatesequenzen auf das Nukleotid genau hergestellt werden, ohne daß hierfür Klonierungsarbeiten notwendig sind. Durch Einführung von Linksequenzen kann man Ribozyme herstellen, die sich über eine zur Linksequenz komplementäre DNA/RNA an eine beliebige chemische Verbindung wie Peptide, Nukleinsäuren, aliphatische Kohlenwasserstoffe, Ester, Ether oder Alkohole koppeln lassen. Wenn diese Verbindung an eine feste Phase gebunden vorliegt, lassen sich Ribozyme selektionieren, die diese Bindung spalten. Diejenigen Ribozyme, die sich selbst von der Bindung an die feste Phase "befreit" haben, können durch reverse Transkription und nachfolgende asymmetrische PCR in einzelsträngige DNA-Moleküle überführt werden. Diese werden dann über die Linksequenz an ein entsprechend modifiziertes Anchor-Oligonukleotid hybridisiert und ligiert. Das verwendete Anchor-Oligonukleotid ist so konstruiert, daß es einen T7-Promoter enthält, über den mit Hilfe der T7-Polymerase wieder das Ribozym erhalten werden kann. Durch die Verwendung einer ungenauen Reversen Transkriptase (z.B. HIV RT) lassen sich zufällige Mutationen einführen. Der Selektionsdruck kann durch immer kürzere Inkubationen erhöht werden, so daß präferentiell Ribozyme mit einer hohen Aktivität amplifiziert werden. Analog lassen sich nach demselben Prinzip auch Ribozyme mit der Fähigkeit selektionieren, eine Bindung zur festen Phase zu vermitteln.

### 8. Verwendung von mit dem erfindungsgemäßen Verfahren produzierten ssDNAs in der Diagnostik (PathoCheck™)

Bei der Diagnostik von Infektionskrankheiten wird üblicherweise häufig ein direkter Erregernachweis z.B. durch PCR verlangt. Besonders in der Transfusionsmedizin ist es wichtig, kontaminierte Blutproben sicher zu erkennen und auszusortieren. Die hierfür üblicherweise eingesetzten serologischen Assays können dies nur dann gewährleisten, wenn die Infektion des Spenders bereits einige Zeit zurückliegt, so daß bereits Antikörper gebildet worden sind. Während eines Fensters von bis zu 12 Wochen (in Extremfällen auch länger) sind beispielsweise bei der HIV-Infektion noch keine Antikörper im Blut nachweisbar, obwohl bereits eine massive Virusreplikation stattfindet. Da eine routinemäßige PCR-Untersuchung aller Proben aus Kostengründen vielerorts kaum machbar ist, wird diese (wenn überhaupt) an Pools von Einzelspenden durchgeführt. Die Problematik dabei ist, daß dadurch die an sich sehr hohe Sensitivität sinkt, da die Menge des für die Analyse eingesetzten Materials nicht beliebig erhöht werden kann. Bei Viruserkrankungen wie HIV, bei denen ein Großteil der Viren extrazellulär vorliegt, kann dies durch eine Konzentrierung der Viren durch Zentrifugation noch einigermaßen ausgeglichen werden, bei vorwiegend zellassoziierten Viren funktioniert das allerdings meist weniger gut. Man kann zwar zunächst DNA oder RNA aus den Blutzellen isolieren, jedoch nur einen Bruchteil davon in der PCR-Reaktion einsetzen, da sonst unspezifische PCR-Produkte überhandnehmen. Daher muss in solchen Fällen eine Vorselektion des zu amplifizierenden Materials durchgeführt werden. Hierfür wird ein einzelsträngiges, mit dem erfindungsgemäßen Verfahren hergestelltes Produkt eingesetzt, das mit einem modifizierten Anchor-Oligonukleotid hergestellt wird. In diesem Fall verwendet man ein Anchor-Oligonukleotid aus zwei getrennten komplementären Strängen, von denen der eine am 5'-Ende modifiziert, z.B. biotinyliert, ist; der andere am 3'-Ende blockiert ist. Nach der Synthese der Virus-Sequenz wird der nichtbiotinylierte Strang durch Waschen mit einer denaturierenden Lösung abgetrennt, so daß eine einzelsträngige Antisense-DNA verbleibt. Diese kann mit dem 5'-biotinylierten Teil-Anchor-Oligonukleotid und einem endständigen Oligonukleotid amplifiziert werden, sofern mehr Material benötigt wird. Von diesem PCR-Produkt ist nur ein Strang blotinyliert, der andere kann durch Denaturierung abgetrennt werden. Diese Antisense-DNA kann nun dafür eingesetzt werden, um virale RNAs oder DNAs aus einem komplexen Gemisch wie einem Zelllysat oder einer Nukleinsäurepräparation anzureichern, indem man diese miteinander hybridisiert, die Hybride an einen Streptavidin-beschichteten Träger (Support) bindet und nicht-hybridisierte Komponenten unter stringenten Bedingungen wegwäscht. In einem zweiten Schritt können die angereicherten RNAs oder DNAs dann mit einer konventionellen PCR mit Primern aus dem nicht-hybridisierten Teil der RNA oder DNA amplifiziert und nachgewiesen werden. Dies kann üblicherweise über Gelelektrophorese der Produkte geschehen oder durch Fluoreszenzanalyse oder durch einen nachgeschalteten ELISA, unter der Voraussetzung, daß ein entsprechend modifizierter Primer verwendet wurde. Vorteile des erfindungsgemäßen Verfahrens sind, daß fast beliebig hohe.Mengen an Ausgangsmaterial eingesetzt werden können, was die Sensitivität der Analyse verbessert, daß auch mehrere Targets gleichzeitig untersucht und bei Verwendung verschieden fluoreszenzmarkierter Primer auch differenziert werden können und daß es für beliebige Pathogene wie Bakterien, Pilze oder Viren einsetzbar ist. Durch die Voranreicherung der zu amplifizierenden Sequenzen werden nebenbei auch Hintergrundprobleme deutlich reduziert. Bei entsprechender Miniaturisierung kann eine große Anzahl verschiedener Pathogene gleichzeitig auf einem Chip getestet werden, wodurch die Analysekosten extrem gesenkt werden können.

### 9. "Gene Profiling" (GPro™)

In der molekularbiologischen Forschung und zunehmend auch in der molekularen Diagnostik wird die Expression bestimmter Gene auf der RNA-Ebene quantitativ untersucht. Standardwerkzeuge hierfür sind der Northern-Blot, das S1-Mapping oder der "Ribonuclease Protection Assay" (RPA), meist in Verbindung mit radioaktiv markierten Sonden. Die vorstehend beschriebenen einzelsträngigen DNAs können auch für diese Aufgabe verwendet werden. Eine vorhergehende Aufreinigung der zu analysierenden RNAs, die meist eine zusätzliche Fehlerquelle darstellt, ist hierzu nicht notwendig. Ähnlich wie bei dem erfindungsgemäßen PathoCheck™-Verfahren werden die zu untersuchenden mRNAs mit einem Überschuss eines modifizierten z.B. biotinylierten Anchor-Oligonukleotids mit genspezifischen einzelsträngigen Antisense-DNAs hybridisiert und an einer z.B. Streptavidin-beschichteten festen Phase immobilisiert. Nach dem Auswaschen aller Proteine, nicht relevanter Nukleinsäuren und sonstiger Verunreinigungen werden die Ziel-mRNAs mit einer Reihe von direkt oder indirekt fluoreszenzmarkierten Splinkersequenzen, welche zu einem anderen Teil dieser mRNAs komplementär sind, nachgewiesen. Durch die Verwendung verschiedener genspezifischer Antisense-DNAs und unterschiedlich markierter Nachweissplinker-Oligonukleotide kann die Expression mehrerer Gene gleichzeitig analysiert werden. Das ganze Verfahren lässt sich ohne großen Aufwand vollständig automatisieren. Sofern die zu analysierenden Gewebe nicht übermäßig viel RNase enthalten, reicht eine Lyse in chaotropen Puffern und/oder Zugabe von RNasin aus, die Integrität der RNAs zu gewährleisten. Wenn maximale Sensitivität wichtiger ist als der gleichzeitige Nachweis verschiedener mRNAs in einem Reaktionsansatz, können anstelle der fluoreszenzbasierten Nachweisreagenzien auch Antikörper eingesetzt werden, welche an ein polyvalentes Sekundärreagenz binden wie ein anti-Maus-Ig-Peroxidase-Polymer. Diese Komplexe werden dann in einer nachgeschalteten Enzymreälction detektiert z.B. durch die beim Umsetzen eines geeigneten Substrats entstehende Chemilumineszenz. Für besonders häufig gemachte Untersuchungen können entsprechende GPro™-Kits mit synthetischen KontrollmRNAs als quantitative Standards bereits fertig konfektioniert werden.

### 10. Allelidentifizierung durch hybridvermittelte Ligation (LIMA™; Ligation mediated Identification of Mutant Alleles)

Besonders in der Pränataldiagnostik von Erbkrankheiten, aber auch zur Bestimmung der individuellen Sensitivität gegenüber verschiedenen Arzneimitteln muss der Genotyp bestimmter Allele festgestellt werden. In der Regel geschieht dies durch eine PCR-Amplifikation des zu untersuchenden Lokus aus der genomischen DNA und anschließende Restriktionsanalyse oder Sequenzierung. Im ersten Fall bedingt dies eine gelelektrophoretische Auftrennung der Restriktionsfragmente, die nicht ohne weiteres automatisierbar ist. Das trifft auch im zweiten Fall zu, sofern nicht mit der Chipsequenzierungsmethode gearbeitet wird, die jedoch noch nicht ausgereift ist. Auch für diesen Aspekt lassen sich erfindungsgemäß hergestellte DNA-Fragmente verwenden. Voraussetzung hierfür ist, daß es sich um bekannte, molekular identifizierte Allele handeln, muss. Ein erfindungsgemäß hergestelltes Anchor-Oligonukleotid wird dann so konstruiert, daß es an eine Genregion hybridisiert, die unmittelbar vor der Mutation liegt. Ein weiteres Oligonukleotid, das ein oder mehrere fluoreszierende Markierungen enthält, hybridisiert an die direkt angrenzende 3'-Region des Gens, so daß die beiden freien Enden der erfindungsgemäß hergestellten Anchor-Oligonukleotid-DNA und des fluoreszenzmarkierten Oligonukleotids bei durchgängiger Hybridbildung direkt nebeneinander zu liegen kommen und miteinander ligiert werden können. Sofern die Sequenz an dieser Stelle abweicht, kommt es nicht zur Anlagerung der Enden und damit auch nicht zur Ligation. Stattdessen kann z.B. ein mit einer anderen Fluoreszenz markiertes Oligonukleotid an die entsprechende mutierte Sequenz binden, wodurch eine andere Markierung an den biotinylierten Anchor ligiert wird. Durch Laseranregung werden die jeweils gebundenen Fluoreszenzfarbstoffe und damit die jeweiligen Allele identifiziert. Zur Erhöhung der Sensitivität des erfindungsgemäßen Verfahrens kann auch in diesem Fall eine asymmetrische PCR vorgeschaltet werden, welche den zu untersuchenden Lokus amplifiziert. Bei einheitlichen Reaktionsbedingungen für die PCR und Hybridisierung ist es möglich, mehrere verschiedene Allele gleichzeitig aus einer Probe zu bestimmen.

### 11. Direkte Interaktionsanalyse von Protein Arrays (LISPA™)

Mit dem Erfolg des "Human Genome Project" steht als eine der nächsten Aufgaben die Klassifizierung der ca. 50.000 menschlichen Gene an. Nicht nur in der Grundlagenfarschung, sondern auch in dem sich rasch fortentwickelnden Gebiet der molekularen Medizin ist es wichtig zu verstehen, was diese Gene tun, wie sie in welchen Situationen miteinander kooperieren, welche Proteine, Peptide oder niedermolekularen Stoffe an welche anderen Proteine binden etc. Ein erster Anhaltspunkt für solche Kooperationen zwischen Proteinen ist meist ein direkter physischer Kontakt der jeweiligen Genprodukte. Um solche Bindungen in vitro auf der Proteinebene untersuchen zu können, benötigt man in der Regel aufgereinigte Proteinpräparationen. Bei 50.000 Proteinen ist dies jedoch nicht ohne weiteres möglich. Man behilft sich daher zumeist mit genetischen Methoden wie z.B. dem sogenannten "Yeast Two Hybrid Screen", um mögliche Interaktionspartner zu identifizieren. So erfolgreich diese Methode bisher auch eingesetzt worden ist, ist sie dennoch extrem artefaktanfällig, umständlich und für die Komplexität der nun anstehenden Aufgabe ungeeignet. Diese Aufgabe kann mit einer Kombination des erfindungsgemäßen Verfahrens, des Sloning™-Verfahrens, und dem EasyPro™-Verfahren in Verbindung mit einem Biochip durchgeführt werden. Mittels einer Automatisierung können die kompletten 50.000 Gene synthetisiert, exprimiert und mit einem geeigneten Tag zur Immobilisierung in Reaktionskammern eines Biochips versehen werden. Für Bindungsstudien mit einem fluoreszenzmarkierten Protein oder einer niedermolekularen chemischen Verbindung ist eine Menge von 10⁷ bis 10⁸ Molekülen üblicherweise ausreichend. In Kavitäten von 100 x 200 x 60 µm können etwa 1 Nanoliter einer Proteinlösung deponiert werden, dies entspricht bei einem 100 kD Protein und einer Konzentration von 5 mg/ml ca. 3 x 10¹⁰ Molekülen. Geht man davon aus, daß die tatsächliche Bindungskapazität pro Kavität bei ca. 1% dieses Werts liegt, ist auch bei relativ großen Proteinen noch ausreichend Material vorhanden. Wenn die einzelnen Kavitäten ca. 30 µm auseinanderliegen, so könnte die gesamte Bibliothek von 50.000 Proteinen auf einem Chip von nur 20 cm² untergebracht werden. Ein Laser mißt die Fluoreszenz in sämtlichen Kavitäten vor und nach der Bindung des fluoreszenzmarkierten Zielmoleküls, woraus man die Stärke der Interaktion berechnt. Kavitäten, in denen nur das Tag präsentiert wird, dienen als unspezifische Kontrolle. Mit Hilfe solcher Proteinarrays lassen sich beispielsweise bislang nicht feststellbare Bindungen von Arzneimitteln an zelluläre Proteine detektieren oder auch komplizierte Signaltransduktionskaskaden nachvollziehen.

### 12. Parallele Analyse von mRNAs mit immobilisierten Nukleinsäure Arrays (PAMINA™)

Einer der Schwerpunkte der modernen Arzneimittelforschung besteht in dem gezielten Eingriff in die Expression einzelner Gene. Dazu muss der Einfluss von neuen Wirkstoffen auf die Expression anderer Gene möglichst umfassend untersucht werden. Bei Signalübertragungsprozessen, der Zelldifferenzierung oder bei krankheitsinduzierten metabolischen Veränderungen wird oft eine ganze Kaskade verschiedener Gene an- oder abgeschaltet. Aufgrund der Komplexität der Genexpression in höheren Organismen ist es aber bis jetzt praktisch nicht möglich, mehr als eine Handvoll Gene gleichzeitig zu analysieren. Mit der Sequenzierung des menschlichen Genoms werden in Zukunft jedoch die Grundvoraussetzungen für eine umfassende parallele Analyse der gesamten Genexpression einer Zelle geschaffen werden. Aus den vorliegenden Sequenzinformationen lassen sich durch computerisierte Sequenzvergleiche zunächst die Regionen in den einzelnen Genen identifizieren, die die geringste Homologie untereinander aufweisen, also den höchsten Grad an Spezifität für das jeweilige Gen. Aus diesen Genabschnitten können genspezifische, einzelsträngige Antisense-Anchor-DNAs abgeleitet werden, die in einem Array auf einem Biochip immobilisiert werden. Die Antisense-Anchor-DNAs können so konzipiert werden, daß die Schmelztemperaturen sämtlicher RNA/DNA-Hybride in einem engen Fenster liegen. Durch Hybridisierung der gesamten RNA, bzw. der polyA+-RNA der zu untersuchenden Zellen an diesen Array unter Bedingungen maximaler Stringenz werden in jeder Kavität des Biochips RNA/DNA-Hybride erzeugt, falls die korrespondierende mRNA exprimiert wird. In einem 2. Schritt werden die immobilisierten Antisense-Anchor-DNAs an dem hybridisierten RNA-Template durch Behandlung mit einer RNaseH Reversen Transkriptase und modifizierten Nukleotiden, die vorzugsweise fluoreszenzmarkiert sind, verlängert. Nach mehreren Waschvorgängen, um nicht inkorporierte Nukleotide abzutrennen, können die cDNA-Reaktionsprodukte analog zu der beschriebenen LISPA™-Technik durch Laserabtastung der einzelnen Kavitäten gemessen werden.

### Ausführungsbeispiele

### 1. Herstellung der Anchor- und Splinkeroligonukleotide

Die Anchor- und Splinker-Oligonukleotide wurden nach dem von Sinha N.D., Biernat J., McManus J., Köster H., *Nucleic Acids Res,* 1984 Jun, 12:11, 4539-57 beschriebenen Standardverfahren hergestellt oder durch eine Synthese in großem Maßstab, die nacheinander geviertelt wurde oder durch Simultansynthese auf Cellulosemembranen.

### 2. Markierung mit einer Modifikation

Modifikationen in den Oligonukleotiden wurden mit Standardverfahren durchgeführt.

### 3. Kopplung an die Matrix

Zu 10 µl Streptavidin-gekoppelteh magnetisierten Beads (MERCK) in einem Gesamtvolumen von 50 µl in 1xTE/1 M NaCl, pH 7.5 wurden 20-200 pmol Biotin-markierte kinasierte Anchor-Oligonukleotide zugesetzt und 30 min bei Raumtemperatur auf einem Roller inkubiert. Anschließend wurden nicht gebundene Anchor-Olicronukleotide durch einen dreimaligen Pufferwechsel von je 500 µl 1xTE, pH 7.5 weggewaschen.

### 3. Erster Ligationsschritt

Die Ligation erfolgte bei 4°C, 16°C, Raumtemperatur bzw. 37°C (Standard 16°C) in einem Volumen von 50 µl in 1 x Ligasepuffer (Boehringer Mannheim) mit 1 bis 5 Einheiten T4 DNA Ligase (Boehringer Mannheim oder New England Biolabs) für 15 bis 60 Minuten. Für die Ligation würden in der Regel 20 pmol phosphoryliertes Anchor-Oligonukleotid eingesetzt. Am 5'-Ende phosphorylierte Splinker-Oligonukleotide wurden in 1,5 bis 5fachem molaren Überschuß zugegeben. Nach der Reaktion wurden Ligase und nicht-ligierte Splinker-Oligonukleotide durch drei Pufferwechsel von je 500 µl 1xTE, pH 7.5 weggewaschen. Danach wurde zu den gewaschenen Beads 40 µl eines Restriktionsmixes gegeben, der das Splinkerspezifische Restriktionsenzym Eco311 in 1,25x Restriktionspuffer (Puffer A von Boehringer Manheim bzw. Puffer 4 von New England Biolabs) enthielt. Danach wurde wie vorstehend beschrieben gewaschen.

### 5. Zweiter Ligationsschritt

Es wurden vier weitere Ligationen mit weiteren Splinker-Oligonukleotiden nach der unter Punkt 4 aufgeführten Vörschrift durchgeführt.

### 6. Transposition

Nach der 5. Ligation wurde nach dem Waschen ein Mix des Anchor-spezifischen Restriktionsenzyms Esp3I bzw. BpiI in den entsprechenden herstellerspezifischen Puffern hinzugefügt und 30 bis 60 Minuten bei 37°C inkubiert. Nach der Reaktion wurde der komplette Mix mitsamt den abgeschnittenen aufligierten Splinker-Oligonukleotiden entfernt, in einem separaten Reaktionsgefäß 15 Minuten bei 65°C hitzebehandelt, um das Restriktionsenzym zu inaktivieren und sodann in ein weiteres Reaktionsgefäß mit entsprechend aufligierten, an magnetisierte Streptavidin-Beads gekoppelte Anchor-Oligonukleotide ligiert.

### 7. Restriktioriskontrolle ligierter Fragmente

Zur Kontrolle der korrekten Größe der geschnittenen Splinker-Oligonukleotide wurde ein 5 µl Aliquot der Reaktion auf einem 18%igen 1xTBE-Polyacrylamidgel aufgetrennt, mit 0,01% SYBR-Gold™ in 1 x TBE für 10 min angefärbt und mit UV-Licht sichtbar gemacht. Auf einem solchen Gel können Längenunterschiede von 1-2 Basen erkannt werden.

## Patentansprüche

1. Verfahren zur Herstellung eines Nukleinsäuremoleküls umfassend die Schritte
a) Bereitstellen eines Oligonukleotids, das hergestellt ist durch die folgenden Schritte:
aa) Kopplung eines Oligonukleotids mit einem Ende an eine feste Matrix, wobei die Kopplung über eine Modifikation erfolgt, und das Oligonukleotid eine Erkennungssequenz für ein TypIIS Restriktionsenzym enthält, welches außerhalb seiner Erkennungssequenz schneidet,
ab) Zugabe eines weiteren, mindestens teilweise doppelsträngigen Oligonukleotids mit einer anderen Erkennungssequenz für ein TypIIS Restriktionsenzym, welches außerhalb seiner Erkennungssequenz schneidet, als in Schritt aa), wobei dieses Oligonukleotid nicht an die Matrix binden kann,
ac) Ligation der Oligonukleotide aus Schritt aa) und ab) in der durch die Blockierung der nicht zu ligierenden Enden festgelegten Orientierung,
ad) Entfernen nicht verbrauchter Reaktanden sowie Enzyme,
ac) Spaltung des Ligationsprodukts aus Schritt ac) mit einem TypIIS Restriktionsenzym, welches außerhalb seiner Erkennungssequenz schneidet, wobei die Spaltung in der Nukleinsäuresequenz des Oligonukleotids aus Schritt ab) stattfindet,
af) Abtrennen des Reaktionsgemisches vom in Schritt ae) erhaltenen verlängerten Oligonukleotid aus Schritt aa),
ag) mindestens einmaliges Wiederholen der Schritte ab) bis af),
b) Bereitstellen eines weiteren Oligonukleotids, das hergestellt ist durch die Schritte:
ba) Kopplung eines Oligonukleotids mit einem Ende an eine feste Matrix, wobei die Kopplung über eine Modifikation erfolgt, und das Oligonukleotid eine Erkennungssequenz für ein TypIIS Restriktionsenzym enthält, welches außerhalb seiner Erkennungssequenz schneidet,
bb) Zugabe eines weiteren, mindestens teilweise doppelsträngigen Oligonukleotids mit einer anderen Erkennungssequenz für ein TypIIS Restriktionsenzym, weiches außerhalb seine Erkennungssequenz schneidet, als in Schritt ba), wobei dieses Oligonukleotid nicht an die Matrix binden kann,
bc) Ligation der Oligonukleotide aus Schritt ba) und bb) in der durch die Blockierung der nicht zu ligierenden Enden festgelegten Orientierung,
bd) Entfernen nicht verbrauchter Reaktanden sowie Enzyme,
bc) Spaltung des Ligationsproduktes aus Schritt bc) mit einem TypIIS Restriktionsenzym, welches außerhalb seine Erkennungssequenz schneidet, wobei die Spaltung im Oligonukleotid von Schritt bb) stattfindet,
bf) Abtrennen des so verlängerten Nukleinsäuremoleküls vom Reaktionsgemisch,
bg) mindestens einmaliges Wiederholen der Schritte bb) bis bf), wobei im Anschluss an die letzte Ligation in Schritt bc) und Entfernen nicht verbrauchter Reaktanden sowie Enzyme das Ligationspordukt mit einem TypIIS Restriktionsenzym geschnitten wird, wobei die Spaltung in Oligonukleotid von Schritt ba) stattfindet,
c) Ligation der Oligonukleotide aus Schritt a) und b) in der durch die Blockierung der nicht zu ligierenden Enden festgelegten Orientierung,
d) Entfernen nicht verbrauchter Reaktanden sowie Enzyme,
e) Spaltung des Ligationsproduktes aus Schritt c) mit einem TypIIS Restriktionsenzym, welches außerhalb seiner Erkennungssequenz schneidet, wobei die Spaltung im Oligonukleotid aus Schritt a) oder b) stattfindet,
f) Abtrennen des so verlängerten Nukleinsäuremoleküls vom Reaktionsgemisch.

2. Verfahren nach Anspruch 1, **dadurch gekennzeichnet, dass** das in Schritt ab) oder bb) eingesetzte Oligonukleotid ein durch das Verfahren nach Anspruch 1 hergestelltes Nukleinsäuremolekül ist.

3. Verfahren nach Anspruch 1 oder 2, wobei nach Schritt ac), bc) oder c) als Schritt ac)', bc)' oder c)' eine Exonuklease- und/oder Phosphatase-Reaktion durchgeführt wird.

4. Verfahren nach Anspruch 3, wobei das Reaktionsgemisch des Schrittes ac)', bc)' oder c)' nach der Reaktion entfernt wird.

5. Verfahren nach einem der Ansprüche 1 bis 3, wobei das Oligonukleotid aus Schritt a), aa) oder ba) an dem Ende, welches nicht an die Matrix gekoppelt ist, einen Teil einer Erkennungssequenz für ein TypIIS Restriktionsenzym enthält, welches außerhalb seiner Erkennungssequenz schneidet, und der andere Teil der Erkennungssequenz für dieses Restriktionsenzym vom Oligonukleotid aus Schritt ab), bb) oder b) stammt.

6. Verfahren nach Anspruch 1 bis 5, wobei die Modifikation ein Biotinrest, ein Digoxigeninrest, ein Fluoresceinisothiocyanatrest, eine Aminoverbindung oder ein Succinylester ist.

7. Verfahren nach einem der Ansprüche 1 bis 6, wobei das Oligonukleotid aus Schritt aa), ba) oder a) und/oder ab), bb) oder b) über einen Loop verfügt.

8. Verfahren nach Anspruch 7, wobei das Oligonukleotid aus Schritt aa), ba) oder a) über eine Modifikation im Loopbereich an die feste Matrix gekoppelt ist.

9. Verfahren nach einem der Ansprüche 1 bis 8, wobei die feste Matrix ein Kügelchen (bead), vorzugsweise aus Glas oder Polystyrol, ein Objektträger, ein DNA Chip, die Vertiefung einer Mikrotiterplatte (well) oder ein Reaktionsröhrchen ist.

10. Verfahren nach einem der Ansprüche 1 bis 9, wobei die feste Matrix einen Streptavidinrest, einen anti-Digoxigenin-Antikörper oder anti-Fluoresceinisothiocyanat-Antikörper umfasst.

11. Verfahren nach einem der Ansprüche 1 bis 10, wobei die Oligonukleotide aus Schritt aa), ba) oder a) und ab), bb) oder b) an ihren zu ligierenden Enden über zueinander komplementäre Einzelstrangüberhänge verfügen.

12. Verfahren nach Anspruch 11, wobei die Einzelstrangüberhänge 1, 2, 3, 4 oder 5 Nukleotide lang sind.

13. Verfahren nach Anspruch 1 bis 12, wobei die verschiedenen TypIIS Restriktionsendonukleasen durch analog schneidende Ribozyme ersetzt werden.

14. Verfahren nach einem der Ansprüche 1 bis 13, wobei das Oligonukleotid in Schritt ab), bb) oder b) ein PCR-Produkt, ein Plasmidvektor, eine Phagen- oder Virus-DNA, ein künstliches Chromosom oder eine weitere synthetische DNA ist.

15. Kit zur Herstellung einer Nukleinsäuresequenz nach dem Verfahren nach einem der Ansprüche 1 bis 14 umfassend
a) eine Bibliothek mit 4, 16, 64, 256 oder 1.024 verschiedenen Oligonukleotiden mit einem Überhang, wobei sich die Oligonukleotide in der Sequenz ihres Überhanges unterscheiden und die Oligonukleotide über eine Modilkation an einem Ende an eine feste Matrix koppelbar sind und das Oligonukleotid eine Erkennungssequenz oder einen Teil einer Erkennungssequenz für ein Typ IIS Restriktionsenzym enthält, welches außerhalb seiner Erkennungssequenz schneidet,
b) eine weitere Bibliothek mit 16, 256, 4.096, 65.536 oder 1.048.576 verschiedenen Oligonukleotiden mit einem Überhang, wobei sich die Oligonukleotide in der Sequenz ihres Überhanges und in der Sequenz des sich daran anschließenden, zur Länge des Überhanges korrespondierenden Bereiches unterscheiden und jedes der Oligonukleotide eine Erkennungssequenz für ein Typ IIS Restriktionsenzym enthält, das außerhalb seiner Erkennungssequenz schneidet und verschieden ist von dem Restriktionsenzym aus a),
c) eine feste Matrix,
d) Reservoire der für die Herstellung des Nukleinsäuremoleküls benötigten Enzyme und/oder Reagenzien,
wobei bei einer Länge des Überhanges von einem Nukleotid die Bibliothek aus a) 4 verschiedene Oligonukleotide und die Bibliothek aus b) 16 verschiedene Oligonukleotide umfasst,
wobei einer Länge des Überhanges von 2 Nukleotiden die Bibliothek aus a) 16 verschiedene Oligonukleotide und die Bibliothek aus b) 256 verschiedene Oligonukleotide umfasst,
wobei bei einer Länge des Überhanges von 3 Nukleotiden die Bibliothek aus a) 64 verschiedene Oligonukleotide und die Bibliothek aus b) 4.096 verschiedene Oligonukleotide umfasst,
wobei bei einer Länge des Überhanges von 4 Nukleotiden die Bibliothek aus a) 256 verschiedene Oligonukleotide und die Bibliothek aus b) 65.536 verschiedene Oligonukleotide umfasst, und
wobei bei einer Länge des Überhanges von 5 Nukleotiden die Bibliothek aus a) 1.024 verschiedene Oligonukleotide und die Bibliothek aus b) 1.048.576 verschiedene Oligonukleotide umfasst.

## Claims

1. A method for the manufacture of a nucleic acid molecule comprising the steps of
a) providing an oligonucleotide which is prepared by the following steps:
aa) coupling of an oligonucleotide with one end to a solid matrix, whereby the coupling occurs through a modification and the oligonucleotide comprises a recognition sequence for a type IIS restriction enzyme which cuts outside its recognition sequence,
ab) adding a further, at least partially double-stranded oligonucleotide having a recognition sequence for a type IIS restriction enzyme which cuts outside its recognition sequence, whereby the recognition sequence is different from the one in step aa) and whereby this oligonucleotide cannot bind to the matrix,
ac) ligating the oligonucleotide of step aa) and ab) in the orientation which is defined by the blocking of the ends not to be ligated,
ad) removing non-spent reactants and enzymes,
ae) cleaving the ligation product of step ac) with a type IIS restriction enzyme which cuts outside of its recognition sequence, whereby the cleavage occurs in the nucleic acid sequence of the oligonucleotide of step ab),
af) separating the reaction mixture from the extended oligonucleotide of step aa) as obtained in step ae),
ag) repeating steps ab) to af) at least one time,
b) providing a further oligonucleotide which is prepared by the steps of:
ba) coupling of an oligonucleotide with one end to a solid matrix, whereby the coupling occurs through a modification, and the oligonucleotide comprises a recognition sequence for a type ITS restriction enzyme which cuts outside its recognition sequence,
bb) adding a further, at least partially double-stranded oligonucleotide having a recognition sequence for a type IIS restriction enzyme which cuts outside its recognition sequence, whereby the recognition sequence is different from the one in step ba) and whereby this oligonucleotide cannot bind to the matrix,
bc) ligating the oligonucleotides of step ba) and bb) in the orientation which is defined by the blocking of the ends not to be ligated,
bd) removing non-spent reactants and enzymes,
be) cleaving the ligation product of step bc) with a type IIS restriction enzyme which cuts outside of its recognition sequence, whereby the cleavage occurs in the oligonucleotide of step bb),
bf) separating the thus extended nucleic acid molecule from the reaction mixture,
bg) repeating steps bb) to bf) at least one time, whereby subsequent to the last ligation in step bc) and removal of non-spent reactants as well as enzymes the ligation product is cut with a type IIS restriction enzyme, whereby the cleavage occurs in the oligonucleotide of step ba),
c) ligating the oligonucleotides of step a) and b) in the orientation defined by the blocking of the ends not to be ligated,
d) removing non-spent reactants and enzymes,
e) cleaving the ligation product of step c) with a type IIS restriction enzyme which cuts outside its recognition sequence, whereby the cleavage occurs in the oilgonucleotide of step a) or b),
f) separating the thus extended nucleic acid molecule from the reaction mixture.

2. The method according to claim 1, **characterised in that** the oligonucleotide used in step ab) or bb) is a nucleic acid molecule manufactured by the method according to claim 1.

3. The method according to claim 1 or 2, whereby subsequent to step ac), bc) or c) an exonuclease and/or phosphatase reaction is performed as step ac)', bc)' or c)'.

4. The method according to claim 3, whereby the reaction mixture of the step ac)', bc)' or c)' is removed after the reaction.

5. The method according to any of claims 1 to 3, whereby the oligonucleotide of step a), aa) or ba) comprises at the end which is not coupled to the matrix, a portion of a recognition sequence for a type IIS restriction enzyme which cuts outside its recognition sequence, and whereby the other portion of the recognition sequence for this restriction enzyme originates from the oligonucleotide of step ab), bb) or b).

6. The method according to claims 1 to 5, whereby the modification is a biotin group, a digoxigenin group, a fluorescein isothiocyanate group, an amino compound or a succinyl ester.

7. The method according to any of claims 1 to 6, whereby the oligonucleotide from step aa), ba) or a) and/or ab), bb) or b) comprises a loop.

8. The method according to claim 7, whereby the oligonucleotide from step aa), ba) or a) is coupled to the solid matrix by a modification in the loop region.

9. The method according to any of claims 1 to 8, whereby the solid matrix is a bead, preferably made of glass or polystyrene, a slide, a DNA chip, the well of a micro titer plate or a reaction tube.

10. The method according to any of claims 1 to 9, whereby the solid matrix comprises a streptavidine group, an anti-digoxigenin antibody or an anti-fluorescein isothiocyanate antibody.

11. The method according to any of claims 1 to 10, whereby the oligonucleotide of step aa), ba) or a) and ab), bb) or b) have, at their ends to be ligated, single-stranded overhangs which are complementary to each other.

12. The method according to claim 11, whereby the single-strand overhangs are 1, 2, 3, 4 or 5 nucleotides in length.

13. The method according to any of claims 1 to 12, whereby the different type IIS restriction endonucleases are replaced by ribozymes cutting in an analogous manner.

14. The method according to any of claims 1 to 13, whereby the oligonucleotide in step ab), bb) or b) is a PCR product, a plasmid vector, a phage or virus DNA, an artificial chromosome or a further synthetic DNA.

15. A kit for the manufacture of a nucleic acid sequence according to the method according to any of claims 1 to 14 comprising
a) a library consisting of 4, 16, 64, 256 or 1,024 different oligonucleotides having an overhang, whereby the oligonucleotides differ in the sequence of their overhangs and the oligonucleotides can be coupled to a solid matrix by a modification present at one end, and the oligonucleotide comprises a recognition sequence or a part of a recognition sequence for a type IIS restriction enzyme which cuts outside its recognition sequence,
b) a further library consisting of 16, 256, 4,096, 65,536 or 1,048,576 different oligonucleotides having an overhang, whereby the oligonucleotides differ in the sequence of their overhangs and in the sequence of the region following thereafter and corresponding in length to the length of the overhang, and each of the oligonucleotides comprises a recognition sequence for a type IIS restriction enzyme which cuts outside its recognition sequence and which is different from the restriction enzyme of a),
c) a solid matrix,
d) reservoirs for the enzymes and/or reagents needed for the manufacture of the nucleic acid molecule,
whereby if the length of the overhang is one nucleotide, the library of a) comprises four different oligonucleotides and the library of b) comprises 16 different oligonucleotides,
whereby if the length of the overhang is two nucleotides, the library of a) comprises 16 different oligonucleotides and the library of b) comprises 256 different oligonucleotides,
whereby if the length of the overhang is three nucleotides, the library of a) comprises 64 different oligonucleotides and the library of b) comprises 4,096 different oligonucleotides,
whereby if the length of the overhang is four nucleotides, the library of a) comprises 256 different oligonucleotides and the library of b) comprises 65,536 different oligonucleotides, and
whereby if the length of the overhang is five nucleotides, the library of a) comprises 1,024 different oligonucleotides and the library of b) comprises 1,048,576 different oligonucleotides.

## Revendications

1. Procédé d'obtention d'une molécule d'acide nucléique, comprenant les étapes
a) mise à disposition d'un oligonucléotide, qui est obtenu par les étapes suivantes :
aa) couplage d'un oligonucléotide par une extrémité sur une matrice solide, sachant que le couplage s'effectue via une modification et que l'oligonucléotide contient un site de reconnaissance pour une enzyme de restriction de type IIS qui coupe à l'extérieur de son site de reconnaissance,
ab) addition d'un autre oligonucléotide au moins en partie double-brin avec un site de reconnaissance, pour une enzyme de restriction type IIS qui coupe à l'extérieur de son site de reconnaissance, autre que dans l'étape aa), sachant que cet oligonucléotide ne peut pas se lier à la matrice,
ac) ligature des oligonucléotides des étapes aa) et ab) dans l'orientation fixée par le blocage des extrémités ne devant pas être jointes par ligature,
ad) élimination des réactifs ainsi que des enzymes non consommés,
ae) clivage du produit de ligature de l'étape ac) avec une enzyme de restriction type IIS qui coupe à l'extérieur de son site de reconnaissance, sachant que le clivage s'effectue dans la séquence nucléique de l'oligonucléotide de l'étape ab),
af) séparation du mélange réactionnel de l'oligonucléotide allongé de l'étape aa) obtenu à l'étape ae),
ag) au moins une répétition des étapes ab) à af),
b) mise à disposition d'un autre oligonucléotide, qui est obtenu par les étapes :
ba) couplage d'un oligonucléotide par une extrémité sur une matrice solide, sachant que le couplage s'effectue via une modification et que l'oligonucléotide contient un site de reconnaissance pour une enzyme de restriction type IIS qui coupe à l'extérieur de son site de reconnaissance,
bb) addition d'un autre oligonucléotide au moins en partie double-brin avec un site de reconnaissance, pour une enzyme de restriction type IIS qui coupe à l'extérieur de son site de reconnaissance, autre que dans l'étape ba), sachant que cet oligonucléotide ne peut pas se lier à la matrice,
bc) ligature des oligonucléotides des étapes ba) et bb) dans l'orientation fixée par le blocage des extrémités ne devant pas être jointes par ligature,
bd) élimination des réactifs ainsi que des enzymes non consommés,
be) clivage du produit de ligature de l'étape bc) avec une enzyme de restriction type IIS qui coupe à l'extérieur de son site de reconnaissance, sachant que le clivage s'effectue dans l'oligonucléotide de l'étape bb),
bf) séparation de la molécule d'acide nucléique ainsi allongée du mélange réactionnel,
bg) au moins une répétition des étapes bb) à bf), sachant qu'après la dernière ligature à l'étape bc) et l'élimination des réactifs non consommés ainsi que des enzymes, le produit de ligature est coupé par une enzyme de restriction type IIS, sachant que le clivage s'effectue dans l'oligonucléotide de l'étape ba),
c) ligature des oligonucléotides des étapes a) et b) dans l'orientation fixée par le blocage des extrémités ne devant pas être jointes par ligature,
d) élimination des réactifs ainsi que des enzymes non consommés,
e) clivage du produit de ligation de l'étape c) avec une enzyme de restriction type IIS qui coupe à l'extérieur de son site de reconnaissance, sachant que la dissociation s'effectue dans l'oligonucléotide de l'étape a) ou b),
f) séparation de la molécule d'acide nucléique ainsi allongée depuis le mélange réactionnel.

2. Procédé selon la revendication 1, **caractérisé en ce que** l'oligonucléotide utilisé dans l'étape ab) ou bb) est une molécule d'acide nucléique préparée d'après le procédé selon la revendication 1.

3. Procédé selon la revendication 1 ou 2, dans lequel, après l'étape ac), bc) ou c), une réaction par exonucléase et/ou phosphatase est effectuée comme étape ac)', bc)' ou c)'.

4. Procédé selon la revendication 3, dans lequel le mélange réactionnel de l'étape ac)', bc)' ou c)' est éliminé après la réaction.

5. Procédé selon l'une des revendications 1 à 3, dans lequel l'oligonucléotide des étapes a), aa) ou ba) contient à l'extrémité qui n'est pas couplée à la matrice une partie d'un site de reconnaissance pour une enzyme de restriction type IIS, qui coupe à l'extérieur de son site de reconnaissance, et dans lequel l'autre partie du site de reconnaissance pour cette enzyme de restriction provient de l'oligonucléotide de l'étape ab), bb) ou b).

6. Procédé selon l'une des revendications 1 à 5, dans lequel la modification est un groupe biotine, un groupe digoxygénine, un groupe fluorescéine isothiocyanate, un composé amine ou un ester de succinyle.

7. Procédé selon l'une des revendications 1 à 6, dans lequel l'oligc nucléotide de l'étape aa), ba) ou a) et/ou ab), bb) ou b) comporte une boucle.

8. Procédé selon la revendication 7, dans lequel l'oligonucléotide de l'étape aa), ba) ou a) est couplé à la matrice solide par une modification dans la zone de la boucle.

9. Procédé selon l'une des revendications 1 à 8, dans lequel la matrice solide est une bille, de préférence en verre ou en polystyrène, une lame, une puce à A.D.N., le puits d'une plaque de microtitration ou une éprouvette.

10. Procédé selon l'une des revendications 1 à 9, dans lequel la matrice solide comprend un groupe streptavidine, un anticorps anti-digoxigénine ou un anticorps anti-(fluorescéine isothiocyanate).

11. Procédé selon l'une des revendications 1 à 10, dans lequel les oligoriucléotides de l'étape aa), ba) ou a) et ab), bb) ou b) comportent à leurs extrémités à joindre par ligature des séquences monobrin complémentaires entre elles.

12. Procédé selon la revendication 11, dans lequel les séquences monobrin ont une longueur de 1, 2, 3, 4 ou 5 nucléotides.

13. Procédé selon l'une des revendications 1 à 12, dans lequel les différentes endonucléases de restriction type IIS, sont remplacées par des ribozymes qui coupent de manière analogue.

14. Procédé selon l'une des revendications 1 à 13, dans lequel l'oligonucléotide de l'étape ab), bb) ou b) est un produit PCR, un vecteur de plasmidique, un ADN phagique ou viral, un chromosome artificiel ou un autre ADN synthétique.

15. Kit de fabrication d'une séquence d'acide nucléique d'après le procédé selon l'une quelconque des revendications 1 à 14, comprenant
a) une bibliothèque avec 4, 16, 64, 256 ou 1.024 oligonucléotides différents avec une séquence débordante, sachant que les oligonucléotides se distinguent par leur séquence débordante et que les oligonucléotides peuvent être couplés à une matrice solide par une modification à une extrémité et que l'oligonucléotide contient un site de reconnaissance ou une partie d'un site de reconnaissance pour un enzyme de restriction type IIS, qui coupe à l'extérieur de son site de reconnaissance,
b) une autre bibliothèque avec 16, 256, 4.096, 65.536 ou 1.048.576 différents oligonucléotides avec une séquence débordante, sachant que les oligonucléotides se distinguent par leur séquence débordante et par la séquence de la zone s'y raccordant, correspondant à la longueur de la séquence débordante, et que chacun des oligonucléotides contient un site de reconnaissance pour une enzyme de restriction type IIS qui coupe à l'extérieur de son site de reconnaissance et qui est différent de l'enzyme de restriction de a),
c) une matrice solide,
d) des réservoirs pour les enzymes et/ou les réactifs nécessaires pour la fabrication de la molécule d'acide nucléique,
sachant que, pour une longueur de séquence débordante d'un nucléotide, la bibliothèque de a) comprend 4 oligonucléotides différents et la bibliothèque de b) 16 oligonucléotides différents,
pour une longueur de séquence débordante de 2 nucléotides, la bibliothèque de a) comprend 16 oligonucléotides différents et la bibliothèque de b) 256 oligonucléotides différents,
sachant que, pour une longueur de séquence débordante de 3 nucléotides, la bibliothèque de a) comprend 64 oligonucléotides différents et la bibliothèque de b) 4.096 oligonucléotides différents,
sachant que, pour une longueur de séquence débordante de 4 nucléotides, la bibliothèque de a) comprend 256 oligonucléotides différents et la bibliothèque de b) 65.536 oligonucléotides différents, et
sachant que, pour une longueur de séquence débordante de 5 nucléotides, la bibliothèque de a) comprend 1.024 oligonucléotides différents et la bibliothèque de b) 1.048.576 aligonucléotides différents.
